(19) Europäisches Patentamt / European Patent Office / Office européen des brevets

(11) **EP 3 801 894 B1**

(12) **FASCICULE DE BREVET EUROPEEN**

(45) Date de publication et mention
de la délivrance du brevet:
**09.08.2023   Bulletin 2023/32**

(21) Numéro de dépôt: **19724491.6**

(22) Date de dépôt: **16.05.2019**

(51) Classification Internationale des Brevets (IPC):
*B01J 29/80* (2006.01)      *B01J 29/72* (2006.01)
*B01D 53/94* (2006.01)      *B01J 29/74* (2006.01)
*B01J 29/76* (2006.01)      *B01J 29/78* (2006.01)
*C01B 39/02* (2006.01)      *C01B 39/48* (2006.01)
*B01J 37/02* (2006.01)      *B01J 35/04* (2006.01)

(52) Classification Coopérative des Brevets (CPC):
**B01J 29/7215; B01D 53/9418; B01J 29/72;
B01J 29/74; B01J 29/7415; B01J 29/76;
B01J 29/7615; B01J 29/78; B01J 29/7815;
B01J 29/80; B01J 35/04; B01J 37/0246;
C01B 39/023; C01B 39/026; C01B 39/48;**   (Cont.)

(86) Numéro de dépôt international:
**PCT/EP2019/062563**

(87) Numéro de publication internationale:
**WO 2019/224090 (28.11.2019 Gazette 2019/48)**

(54) **CATALYSEUR COMPRENANT UN MELANGE D'UNE ZEOLITHE DE TYPE STRUCTURAL AFX ET D'UNE ZEOLITHE DE TYPE STRUCTURAL BEA ET AU MOINS UN METAL DE TRANSITION POUR LA REDUCTION SELECTIVE DE NOX**

KATALYSATOR MIT EINER MISCHUNG AUS EINEM ZEOLITH MIT AFX-STRUKTUR UND EINEM ZEOLITH MIT BEA-STRUKTUR UND MINDESTENS EINEM ÜBERGANGSMETALL ZUR SELEKTIVEN REDUKTION VON NOX

CATALYST COMPRISING A MIXTURE OF AN AFX-STRUCTURE ZEOLITE AND A BEA-STRUCTURE ZEOLITE AND AT LEAST ONE TRANSITION METAL FOR SELECTIVE REDUCTION OF NOX

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **24.05.2018   FR 1854382**

(43) Date de publication de la demande:
**14.04.2021   Bulletin 2021/15**

(73) Titulaire: **IFP Energies nouvelles
92852 Rueil-Malmaison (FR)**

(72) Inventeurs:
• **BERTHOUT, David
92852 RUEIL-MALMAION (FR)**
• **HARBUZARU, Bogdan
92852 RUEIL-MALMAISON (FR)**
• **LLIDO, Eric
92852 RUEIL-MALMAISON (FR)**

(74) Mandataire: **IFP Energies nouvelles
Département Propriété Industrielle
Rond Point de l'échangeur de Solaize
BP3
69360 Solaize (FR)**

(56) Documents cités:
**WO-A1-2016/205509      WO-A1-2017/087385
WO-A1-2017/202495      US-A1- 2016 096 169**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **MARTÍN NURIA ET AL: "Cage-based small-pore catalysts for NH3-SCR prepared by combining bulky organic structure directing agents with modified zeolites as reagents", APPLIED CATALYSIS B: ENVIRONMENTAL, ELSEVIER, AMSTERDAM, NL, vol. 217, 29 mai 2017 (2017-05-29), pages 125-136, XP085112832, ISSN: 0926-3373, DOI: 10.1016/J.APCATB.2017.05.082**
- **DUSTIN W FICKEL ET AL: "The ammonia selective catalytic reduction activity of copper-exchanged small-pore zeolites", APPLIED CATALYSIS B: ENVIRONMENTAL, ELSEVIER, AMSTERDAM, NL, vol. 102, no. 3, 9 décembre 2010 (2010-12-09), pages 441-448, XP028139896, ISSN: 0926-3373, DOI: 10.1016/J.APCATB.2010.12.022 [extrait le 2010-12-16] cité dans la demande**
- **WANG AIYONG ET AL: "NH3-SCR on Cu, Fe and Cu+Fe exchanged beta and SSZ-13 catalysts: Hydrothermal aging and propylene poisoning effects", CATALYSIS TODAY, vol. 320, 7 octobre 2017 (2017-10-07), pages 91-99, XP085513181, ISSN: 0920-5861, DOI: 10.1016/J.CATTOD.2017.09.061**
- **XIAOJIAO LIU ET AL: "Ammonia selective catalytic reduction of NO over Ce-Fe/Cu-SSZ-13 catalysts", RSC ADVANCES, vol. 5, no. 104, 1 janvier 2015 (2015-01-01), pages 85453-85459, XP055552933, DOI: 10.1039/C5RA16072C**

(52) Classification Coopérative des Brevets (CPC):
(Cont.)
B01D 2251/202; B01D 2251/2062;
B01D 2253/1085; B01D 2255/20761;
B01D 2255/502; B01D 2255/9205; B01J 2229/186

**Description**

**DOMAINE TECHNIQUE DE L'INVENTION**

L'invention a pour objet un procédé de préparation d'un catalyseur à base d'un mélange intime d'une zéolithe de type structural AFX et d'une zéolithe de type structural BEA, et d'au moins un métal de transition, le catalyseur, et son utilisation pour la réduction sélective catalytique des NOx en présence d'un réducteur, en particulier sur les moteurs à combustion interne.

**ART ANTÉRIEUR**

Les émissions d'oxydes d'azote (NOx) qui résultent de la combustion de combustibles fossiles sont une préoccupation majeure pour la société. Des normes de plus en plus sévères sont mises en place par les instances gouvernementales afin de limiter l'impact des émissions issues de la combustion sur l'environnement et sur la santé. Pour les véhicules légers en Europe dans le cadre de la réglementation Euro 6c, les émissions de NOx et de particules doivent atteindre un niveau très bas pour l'ensemble des conditions de fonctionnement. Le nouveau cycle de conduite WLTC (Worldwide harmonized Light vehicles Test Cycle) et la réglementation des émissions en conduite réelle (RDE) associée aux facteurs de conformité, exige le développement d'un système de dépollution hautement efficaces pour atteindre ces objectifs. La réduction catalytique sélective, désignée par l'acronyme anglo-saxon « SCR » pour « Selective Catalytic Réduction », apparait comme une technologie efficace pour éliminer les oxydes d'azote dans les gaz d'échappement riches en oxygène, typiques des moteur Diesel et à allumage commandé en mélange pauvre. La réduction catalytique sélective est réalisé grâce à un réducteur, généralement l'ammoniac, et peut ainsi être désignée par $NH_3$-SCR. L'ammoniac ($NH_3$) impliqué dans le processus SCR est généralement généré via la décomposition d'une la solution aqueuse d'urée (AdBlue ou DEF), et produit $N_2$ et $H_2O$ lors de la réaction avec NOx.

Les zéolithes échangées avec des métaux de transitions sont notamment utilisées comme catalyseurs pour les applications $NH_3$-SCR, dans les transports. Les zéolithes à petit pores, en particulier les chabazites échangées au cuivre, sont particulièrement adaptées. Elles existent commercialement sous la forme silicoaluminophosphate Cu-SAPO-34 et aluminosilicates Cu-SSZ-13 (ou Cu-SSZ-62). Leur tenue hydrothermale et leur efficacité de conversion des NOx en font les références actuelles. Cependant, les normes étant de plus en plus contraignantes, les performances des catalyseurs doivent encore être améliorées.

L'utilisation des zéolithes de type structural AFX pour les applications $NH_3$-SCR est connue, mais peu de travaux évaluent l'efficacité de catalyseurs mettant en oeuvre cette zéolithe.

Fickel et al. (Fickel, D. W., & Lobo, R. F. (2009), The Journal of Physical Chemistry C, 114(3), 1633-1640) étudie l'utilisation d'une SSZ-16 (type structural AFX) échangée au cuivre pour l'élimination des NOx. Cette zéolithe est synthétisée conformément au brevet US 5,194,235, dans lequel le cuivre est introduit par échange en utilisant du sulfate de cuivre(II) à 80°C pendant 1h. Des résultats récents (Fickel, D. W., D'Addio, E., Lauterbach, J. A., & Lobo, R. F. (2011), 102(3), 441-448) montrent une excellente conversion et une bonne tenue hydrothermale pour un chargement à 3,78% poids en cuivre.

Des travaux sur la synthèse de zéolithes de type structural AFX ont été effectués avec différents agents structuraux (Lobo, R. F., Zones, S. I., & Medrud, R. C. (1996), Chemistry of materials, 8(10), 2409-2411) ainsi que des travaux d'optimisation de la synthèse (Hrabanek, P., Zikanova, A., Supinkova, T., Drahokoupil, J., Fila, V., Lhotka, M., Bernauer, B. (2016), Microporous and Mesoporous Materials, 228, 107-115).

Wang et al. (Wang, D. et al., CrystEngComm., (2016), 18(6), 1000-1008) ont étudié le remplacement de l'agent structurant TMHD par un mélange TEA-TMA pour la formation de SAPO-56 et obtiennent des phases non désirées SAPO-34 et SAPO-20. L'incorporation de métaux de transition n'est pas abordée.

La demande US 2016/0137518 décrit une zéolithe AFX quasi-pure, sa synthèse à partir de sources de silice et d'alumine en présence d'un agent structurant de type 1,3-Bis(1-adamantyl)imidazolium hydroxide, la préparation d'un catalyseur à base de la zéolithe AFX échangée avec un métal de transition et son utilisation pour des applications $NH_3$-SCR. Aucune forme particulière de zéolithe AFX n'est évoquée.

Plus récemment, la demande US 2018/0093259 présente la synthèse de zéolithes à petits pores, comme la zéolithe de type structural AFX, à partir de zéolithe de type FAU en présence d'un structurant, comme le 1,3-bis(1-adamantyl)imidazolium hydroxide et d'une source de métal alcalino-terreux. Elle présente également des applications de la zéolithe de type structural AFX obtenue, en particulier l'utilisation de cette zéolithe comme catalyseur de la réduction de NOx, après échange avec un métal comme le fer. Parallèlement, la demande US 2016/0096169A1 présente l'utilisation dans la conversion des NOx, d'un catalyseur à base d'une zéolithe de type structural AFX ayant un rapport Si/Al de 15 à 50 échangée avec un métal, la zéolithe AFX étant obtenue à partir d'un agent structurant de type 1,3-Bis(1-adamantyl)imidazolium hydroxide. Les résultats obtenus, dans la conversion des NOx, montrent en particulier une sélectivité des catalyseurs préparés selon les demandes US 2018/0093259 et US 2016/0096169 vers le protoxyde d'azote ne dépassant pas 20 ppm.

Le document JP 2014-148441 décrit la synthèse d'un solide apparenté à une zéolithe AFX, en particulier d'une SAPO-56 comprenant du cuivre utilisable pour la réduction des $NO_x$. Le solide est synthétisé, puis ajouté à un mélange comprenant un alcool et un sel de cuivre, le tout étant calciné. Le cuivre est donc ajouté après la formation du solide SAPO apparenté à la zéolithe de type structural AFX. Ce solide échangé semble présenter une résistance accrue à la présence d'eau.

Ogura et al. (Bull. Chem. Soc. Jpn. 2018, 91, 355-361) montrent la très bonne activité d'une zéolithe de type SSZ-16 échangée au cuivre par rapport à d'autres structures zéolithiques et ce même après vieillissement hydrothermal.

WO 2017/080722 présente une synthèse directe d'une zéolithe comprenant du cuivre. Cette synthèse impose de partir d'une zéolithe de type structural FAU et d'utiliser un agent complexant TEPA et un élément $M(OH)_x$ pour aboutir à différents types de zéolithes, principalement de type CHA. Des zéolithes de type ANA, ABW, PHI et GME sont également WO2017202495 A1 divulgue un procédé de préparation d'une zéolithe de type structural AFX à partir d'un mélange aqueux comprenant une zéolithe de type structural FAU, de la silice colloïdale , et du dihydroxyde de 1,6-bis(méthylpiperidinium)hexane.

L'article scientifique par Nuria Martin et al., dans Applied Catalysis B: Environmental 217 (2017) 125-136 divulgue une méthode de préparation d'un catalyseur à base de zéolithe AFX et de cuivre, comprenant une étape de préparation d'une zéolithe AFX à partir d'un mélange comprenant entre autres une zéolithe FAU et un dérivé diquaternaire du dimethylpyrrolidinium, suivie par une étape d'échange ionique avec une solution de cuivre.

La demanderesse a découvert qu'un catalyseur à base d'un mélange intime d'une zéolithe de type structural AFX et d'une zéolithe de type structural BEA, préparé selon un mode de synthèse particulier et d'au moins un métal de transition, en particulier du cuivre, présentait des performances intéressantes de conversion des $NO_x$ et de sélectivité vers $N_2O$. Les performances de conversion des NOx, en particulier à basse température (T<250°C), sont notamment supérieures à celles obtenues avec des catalyseurs de l'art antérieur, tels que les catalyseurs à base de zéolithe de type structural AFX échangée au cuivre, tout en conservant une bonne sélectivité vers le protoxyde d'azote $N_2O$.

## RESUME DE L'INVENTION

La présente invention concerne un procédé de préparation d'un catalyseur comprenant un mélange de zéolithes de type structural AFX et de type structural BEA, et au moins un métal de transition, comprenant au moins les étapes suivantes : i) le mélange en milieu aqueux, d'une zéolithe FAU ayant un rapport molaire $SiO_2/Al_2O_3$ compris entre 30 et 100 avec au moins une zéolithe de type structural FAU ayant un ratio molaire $SiO_2/Al_2O_3$ compris entre 2 et 30 (borne supérieure exclue), et où le paramètre mathématique, $P_{ze}$, correspondant au pourcentage massique de la zéolithe FAU de rapport molaire $SiO_2/Al_2O_3$ compris entre 30 et 100, sous sa forme anhydre (exprimé en %) dans le mélange de zéolithes FAU, multiplié par le rapport molaire $SiO_2/Al_2O_3$ de ladite même zéolithe FAU de rapport molaire $SiO_2/Al_2O_3$ compris entre 30 et 100, est tel que : $3250 < P_{ze} < 7200$, d'au moins un composé organique azoté R, choisi parmi le dihydroxyde de 1,5-bis(méthylpiperidinium)pentane, le dihydroxyde de 1,6-bis(méthylpiperidinium)hexane, le dihydroxyde de 1,7-bis(méthylpiperidinium) heptane et leurs mélanges, au moins une source d'au moins un métal alcalin et/ou métal alcalino-terreux M de valence n, n étant un nombre entier supérieur ou égal à 1, le mélange réactionnel présentant la composition molaire suivante :

| | |
|---|---|
| $(SiO_{2(FAU)})/(Al_2O_{3(FAU)})$ | compris entre 30 et 80, |
| $H_2O/(SiO_{2(FAU)})$ | compris entre 1 et 100, |
| $R/(SiO_{2(FAU)})$ | compris entre 0,01 et 0,6, |
| $M_{2/n}O/(SiO_{2(FAU)})$ | compris entre 0,005 et 0,45, |

dans laquelle $SiO_{2\ (FAU)}$ est la quantité molaire de $SiO_2$ apportée par l'ensemble des zéolithes de type structural FAU introduites dans le mélange, $Al_2O_{3\ (FAU)}$ est la quantité molaire de $Al_2O_3$ apportée par l'ensemble des zéolithes de type structural FAU introduites dans le mélange, $H_2O$ la quantité molaire d'eau présente dans le mélange réactionnel, R la quantité molaire dudit composé organique azoté, $M_{2/n}O$ étant la quantité molaire de $M_{2/n}O$ apportée par l'ensemble des zéolithes FAU et par la source de métal alcalin et/ou de métal alcalino-terreux,

jusqu'à l'obtention d'un gel précurseur;

ii) le traitement hydrothermal dudit gel précurseur obtenu à l'issue de l'étape i) à une température comprise entre 120°C et 220°C, pendant une durée comprise entre 12 heures et 15 jours, pour obtenir une phase solide cristallisée, dite « solide » ;

iii) au moins un échange ionique comprenant la mise en contact du solide obtenu à l'issue de l'étape précédente, avec au moins une solution comprenant au moins une espèce apte à libérer un métal de transition en solution sous forme réactive sous agitation à température ambiante pendant une durée comprise entre 1 heure et 2 jours , le métal de transition libéré dans la solution de l'étape iii) étant sélectionné dans le groupe formé des éléments suivants : Ti, V, Mn, Mo, Fe, Co, Cu, Cr, Zn, Nb, Ce, Zr, Rh, Pd, Pt, Au, W, Ag, et la teneur en métal de transition étant comprise entre 0,5 à 6% massique, par rapport à la masse totale du catalyseur final, sous sa forme anhydre ;

iv) le traitement thermique par séchage du solide obtenu à l'issue de l'étape précédente à une température comprise entre 20 et 150°C pendant une durée compris entre 2 et 24 heures, suivi d'une calcination sous flux de d'air à une température comprise entre 450 et 700°C pendant une durée comprise entre 2 et 20 heures.

La présente invention concerne également un catalyseur comprenant un matériau composite contenant un mélange intime d'une zéolithe de type AFX et d'une zéolithe de type BEA, et au moins un métal de transition, présentant une structure zéolithique comprenant :

- entre 30 et 90% massique, de préférence entre 40 et 90% massique de zéolithe de type structural AFX, par rapport à la masse totale dudit catalyseur sous sa forme anhydre ;
- entre 10 et 70% massique, de préférence entre 10 et 60% massique de zéolithe de type structural BEA, par rapport à la masse totale dudit catalyseur sous sa forme anhydre ; le catalyseur présentant un rapport molaire $SiO2/Al2O3$ compris entre 2 et 100, de préférence compris entre 4 et 90 et plus préférentiellement entre 6 et 80,

dans lequel ledit métal de transition est sélectionné dans le groupe formé des éléments suivants : Ti, V, Mn, Mo, Fe, Co, Cu, Cr, Zn, Nb, Ce, Zr, Rh, Pd, Pt, Au, W, Ag, de préférence Cu, Fe, Nb, Ce, Mn, de manière très préférée le fer, le cuivre ou leur mélange,
dans lequel la teneur totale en métal de transition est comprise entre 0,5 à 6% massique, de préférence entre 0,5 et 5% massique, et de façon encore plus préférée entre 1 et 4% massique, par rapport à la masse totale du catalyseur final, sous sa forme anhydre.

La présente invention concerne également l'utilisation du catalyseur précédent ou préparé par le procédé décrit précédemment, pour la réduction sélective de $NO_x$ par un réducteur pour la réduction sélective catalytique des NOx tel que $NH_3$ ou $H_2$.

L'intérêt de la présente invention réside dans le catalyseur particulier, à base d'un matériau composite contenant un mélange intime d'une zéolithe de type structural AFX et une zéolithe de type structural BEA, et d'un métal de transition, en particulier du cuivre. Le catalyseur selon l'invention présente en effet des propriétés améliorées par rapport aux catalyseurs de l'art antérieur. En particulier, l'utilisation du catalyseur selon l'invention ou préparé selon le procédé de l'invention permet d'obtenir des températures d'amorçage plus faibles pour la réaction de conversion des NOx et une meilleure conversion des NOx sur l'ensemble de la gamme de température de fonctionnement (150°C- 600°C), tout en conservant une bonne sélectivité en $N_2O$.

## DESCRIPTION DÉTAILLÉE DE L'INVENTION

Conformément à l'invention, on définit un paramètre $P_{ze}$ pour les zéolithes FAU ayant un rapport molaire $SiO_2/Al_2O_3$ compris entre 30 et 100 présentes dans le mélange de zéolithes FAU de départ, comme le produit mathématique entre le pourcentage massique de la zéolithe FAU de rapport molaire $SiO_2/Al_2O_3$ compris entre 30 et 100, sous sa forme anhydre (exprimé en %) dans le mélange de zéolithes FAU de départ, et son rapport molaire $SiO_2/Al_2O_3$, la somme des pourcentages massiques de chacune des zéolithes FAU sous la forme anhydre dans le mélange de départ étant égale à 100. Par exemple, lorsqu'on part d'un mélange de deux zéolithes FAU, ledit paramètre $P_{ze}$ pour la zéolithe ayant un rapport molaire $SiO_2/Al_2O_3$ compris entre 30 et 100 correspond au pourcentage (exprimé en %) de ladite zéolithe FAU ayant un rapport molaire $SiO_2/Al_2O_3$ compris entre 30 et 100 sous sa forme anhydre dans le mélange de zéolithes FAU de départ multiplié par le ratio molaire $SiO_2/Al_2O_3$ de ladite zéolithe FAU ayant un rapport molaire $SiO_2/Al_2O_3$ compris entre 30 et 100.

Selon l'invention, les termes « gel », « gel précurseur » sont synonymes et correspondent au mélange réactionnel homogène obtenu en fin d'étape i) du procédé selon l'invention.

Selon la présente invention, l'expression « compris entre ... et ... » signifie que les valeurs aux bornes de l'intervalle sont incluses dans la gamme de valeurs décrite. Si tel n'était pas le cas et que les valeurs aux bornes n'étaient pas incluses dans la gamme décrite, une telle précision sera apportée par la présente invention.

La présente invention concerne, en particulier, un procédé de préparation d'un catalyseur comprenant un mélange de zéolithes de type structural AFX et de type structural BEA, et au moins un métal de transition, comprenant au moins les étapes suivantes :
i) le mélange en milieu aqueux, d'une zéolithe FAU ayant un rapport molaire $SiO_2/Al_2O_3$ compris entre 30 et 100 avec au moins une zéolithe de type structural FAU ayant un ratio molaire $SiO_2/Al_2O_3$ compris entre 2 et 30 (borne supérieure exclue), et où le paramètre mathématique, $P_{ze}$, correspondant au pourcentage massique de la zéolithe FAU de rapport molaire $SiO_2/Al_2O_3$ compris entre 30 et 100, sous sa forme anhydre (exprimé en %) dans le mélange de zéolithes FAU, multiplié par le rapport molaire $SiO2/Al2O3$ de ladite même zéolithe FAU de rapport molaire $SiO_2/Al_2O_3$ compris entre 30 et 100, est tel que : $3250 < P_{ze} < 7200$, de préférence $3350 < P_{ze} < 7100$, d'au moins un composé organique azoté R, choisi parmi le dihydroxyde de 1,5-bis(méthylpiperidinium)pentane, le dihydroxyde de 1,6-bis(méthylpiperidinium)hexane, le dihydroxyde de 1,7-bis(méthylpiperidinium) heptane et leurs mélanges, au moins une source d'au moins un métal alcalin et/ou métal alcalino-terreux M de valence n, n étant un nombre entier supérieur ou égal à 1, le mélange réactionnel présentant la composition molaire suivante :

| | |
|---|---|
| $(SiO_{2(FAU)})/(Al_2O_{3(FAU)})$ | compris entre 30 et 80, de préférence entre 32 et 70 |
| $H_2O/(SiO_{2(FAU)})$ | compris entre 1 et 100, de préférence entre 5 et 60 |
| $R/(SiO_{2(FAU)})$ | compris entre 0,01 et 0,6, de préférence entre 0,05 et 0,5 |
| $M_{2/n}O/(SiO_{2(FAU)})$ | compris entre 0,005 et 0,45, de préférence entre 0,01 et 0,25 |

dans laquelle $SiO_{2\ (FAU)}$ est la quantité molaire de $SiO_2$ apportée par l'ensemble des zéolithes de type structural FAU introduites dans le mélange, $Al_2O_{3\ (FAU)}$ est la quantité molaire de $Al_2O_3$ apportée par l'ensemble des zéolithes de type structural FAU introduites dans le mélange, $H_2O$ la quantité molaire d'eau présente dans le mélange réactionnel, R la quantité molaire dudit composé organique azoté, $M_{2/n}O$ étant la quantité molaire de $M_{2/n}O$ apportée par l'ensemble des zéolithes FAU et par la source de métal alcalin et/ou de métal alcalino-terreux,
jusqu'à l'obtention d'un gel précurseur;
ii) le traitement hydrothermal dudit gel précurseur obtenu à l'issue de l'étape i) à une température comprise entre 120°C et 220°C, pendant une durée comprise entre 12 heures et 15 jours pour obtenir une phase solide cristallisée, dite « solide » ;
iii) au moins un échange ionique comprenant la mise en contact dudit solide obtenu à l'issue de l'étape précédente, avec au moins une solution comprenant au moins une espèce apte à libérer un métal de transition, en particulier le cuivre, en solution sous forme réactive sous agitation à température ambiante pendant une durée comprise entre 1 heure et 2 jours , le métal de transition libéré dans la solution de l'étape iii) étant sélectionné dans le groupe formé des éléments suivants : Ti, V, Mn, Mo, Fe, Co, Cu, Cr, Zn, Nb, Ce, Zr, Rh, Pd, Pt, Au, W, Ag, et la teneur en métal de transition étant comprise entre 0,5 à 6% massique, par rapport à la masse totale du catalyseur final, sous sa forme anhydre ;
iv) le traitement thermique par séchage du solide obtenu à l'issue de l'étape précédente à une température comprise entre 20 et 150°C pendant une durée compris entre 2 et 24 heures, suivi d'une calcination sous flux d'air à une température comprise entre 450 et 700°C pendant une durée comprise entre 2 et 20 heures ;les étapes iii) et iv) pouvant avantageusement être interverties, et éventuellement répétées si besoin.
La présente invention concerne également le catalyseur comprenant 2. un matériau composite contenant un mélange intime de zéolithes de type structural AFX et de type structural BEA et au moins un métal de transition, comme défini à la revendication 13.
L'invention concerne enfin l'utilisation d'un catalyseur selon l'invention pour la réduction sélective catalytique des NOx en présence d'un réducteur.

## Le catalyseur

Le catalyseur selon l'invention comprend au moins un mélange intime d'une zéolithe de type AFX et d'une zéolithe de type BEA, et au moins un métal de transition additionnel, de préférence le cuivre.
Le catalyseur selon l'invention présente une structure zéolithique comprenant :

- entre 30 et 90% massique, de préférence entre 40 et 90% massique de zéolithe de type structural AFX, par rapport à la masse totale dudit catalyseur sous sa forme anhydre ;
- entre 10 et 70% massique, de préférence entre 10 et 60% massique de zéolithe de type structural BEA, par rapport à la masse totale dudit catalyseur sous sa forme anhydre.

De préférence, le rapport massique (AFX/BEA) de la zéolithe AFX par rapport à la zéolithe BEA, dans le catalyseur selon l'invention, est compris entre 0,4 et 9, préférentiellement entre 0,5 et 7,5 et plus préférentiellement encore entre 1 et 4.
Avantageusement, la zéolithe de type structural BEA du catalyseur selon l'invention est de préférence une zéolithe beta contenant un mélange contenant entre 35 et 45% massique, de préférence 40% massique, de polymorphe A et entre 55 et 65% massique, de préférence 60% massique, de polymorphe B.

Le catalyseur selon l'invention présente un rapport molaire $SiO_2/Al_2O_3$ compris entre 2 et 100, de préférence compris entre 4 et 90 et plus préférentiellement entre 6 et 80.

Selon l'invention, les termes « matériau composite », « matériau composite zéolithique » et « matériau aluminosilicate composite » sont synonymes et employés indifféremment pour désigner le matériau solide composé d'un mélange intime de zéolithes de type structural AFX et de type structural BEA. Le matériau composite du catalyseur selon l'invention est obtenu par synthèse directe, c'est-à-dire en une seule étape réactionnelle, à l'issue de l'étape ii) du procédé de préparation selon l'invention. Le matériau aluminosilicate composite, contenant un mélange intime des zéolithes AFX et BEA, ne résulte pas d'un simple mélange mécanique d'au moins une zéolithe de type structural AFX et d'au moins une zéolithe de type structural BEA.

Selon l'invention, le métal ou les métaux de transition compris dans le catalyseur est(sont) sélectionné(s) parmi les éléments issus du groupe formé par les éléments des groupes 3 à 12 du tableau périodique des éléments incluant les lanthanides. En particulier, le métal ou les métaux de transition compris dans le catalyseur est (sont) sélectionné(s) dans le groupe formé des éléments suivants : Ti, V, Mn, Mo, Fe, Co, Cu, Cr, Zn, Nb, Ce, Zr, Rh, Pd, Pt, Au, W, Ag.

De préférence, le catalyseur selon l'invention comprend du cuivre, seul ou associé avec au moins un autre métal de transition, choisi dans le groupe des éléments listés précédemment ; en particulier Fe, Nb, Ce, Mn. De manière préférée, le métal de transition est le fer, le cuivre ou leur mélange.

Selon l'invention, la teneur totale des métaux de transition est comprise entre 0,5 à 6% massique, de préférence entre 0,5 et 5% massique, et de façon encore plus préférée entre 1 et 4% massique, par rapport à la masse totale du catalyseur final, sous sa forme anhydre.

Avantageusement, le catalyseur défini précédemment est susceptible d'être obtenu ou est directement obtenu par le procédé de préparation selon l'invention et décrit ci-après.

Selon un mode de réalisation de l'invention, le catalyseur ne contient que du cuivre comme métal de transition, la teneur massique en cuivre se situant entre 0,5 et 6%, de préférence entre 0,5 et 5%, préférablement entre 1 et 4% et de manière encore plus préférée entre 1,5 et 3,5% par rapport à la masse totale du catalyseur final sous sa forme anhydre.

Selon un autre mode de réalisation de l'invention, le catalyseur comprend du cuivre et un autre métal de transition, de préférence Fe, Nb, Ce, Mn. Dans ce mode de réalisation, la teneur en cuivre du catalyseur se situe entre 0,05 et 2% massique, de préférence 0,5 et 2% massique alors que celle de l'autre métal de transition se situe préférablement entre 1 et 4% massique, les teneurs en métaux de transition étant données en pourcentage massiques par rapport à la masse totale du catalyseur final sous sa forme anhydre.

Selon un troisième mode de réalisation de l'invention, le catalyseur ne contient que du fer comme métal de transition, la teneur massique en fer se situant entre 0,5 et 5%, de préférence entre 1 et 4% et préférablement entre 1,5 et 3,5% par rapport à la masse totale du catalyseur final sous sa forme anhydre.

Selon un autre mode particulier de réalisation de l'invention, le catalyseur comprend du fer et un autre métal de transition, de préférence Cu, Nb, Ce, Mn. Dans ce mode de réalisation, la teneur en fer du catalyseur se situe entre 0,05 et 2% massique, de préférence entre 0,5 et 2% massique alors que celle de l'autre métal de transition se situe préférablement entre 1 et 4% massique, les teneurs en métaux de transition étant données en pourcentage massiques par rapport à la masse totale du catalyseur final sous sa forme anhydre.

Le catalyseur selon l'invention peut également comprendre d'autres éléments, comme par exemple des métaux alcalins et/ou alcalino-terreux, par exemple le sodium, provenant notamment de la synthèse, en particulier des composés du milieu réactionnel de l'étape i) du procédé de préparation dudit catalyseur.

**Procédé de préparation du catalyseur**

**Etape i) de mélange**

Le procédé de préparation selon l'invention comprend une étape i) de mélange en milieu aqueux, d'une zéolithe FAU ayant un rapport molaire $SiO_2/Al_2O_3$ compris entre 30 et 100, avec au moins une zéolithe de type structural FAU ayant un ratio molaire $SiO_2/Al_2O_3$ compris entre 2 et 30 (borne supérieure exclue), et où $3250 < P_{ze} < 7200$ de préférence $3350 < P_{ze} < 7100$, d'au moins un composé organique azoté R choisi parmi le dihydroxyde de 1,5-bis(méthylpiperidinium)pentane, le dihydroxyde de 1,6-bis(méthylpiperidinium)hexane, le dihydroxyde de 1,7-bis(méthylpiperidinium) heptane et leurs mélanges, au moins une source d'au moins un métal alcalin et/ou métal alcalino-terreux M de valence n, n étant un nombre entier supérieur ou égal à 1, le mélange réactionnel présentant la composition molaire suivante :

| | |
|---|---|
| $(SiO_{2(FAU)})/(Al_2O_{3(FAU)})$ | compris entre 30 et 80, de préférence entre 32 et 70 |
| $H_2O/(SiO_{2(FAU)})$ | compris entre 1 et 100, de préférence entre 5 et 60 |
| $R/(SiO_{2(FAU)})$ | compris entre 0,01 et 0,6, de préférence entre 0,05 et 0,5 |

(suite)

$$M_{2/n}O/(SiO_{2(FAU)}) \qquad \text{compris entre 0,005 et 0,45, de préférence entre 0,01 et 0,25}$$

dans laquelle $SiO_{2\,(FAU)}$ est la quantité molaire de $SiO_2$ apportée par l'ensemble des zéolithes de type structural FAU introduites dans le mélange, $Al_2O_{3\,(FAU)}$ est la quantité molaire de $Al_2O_3$ apportée par l'ensemble des zéolithes de type structural FAU introduites dans le mélange, $H_2O$ la quantité molaire d'eau présente dans le mélange réactionnel, R la quantité molaire dudit composé organique azoté, $M_{2/n}O$ étant la quantité molaire de $M_{2/n}O$ apportée par l'ensemble des zéolithes FAU et par la source de métal alcalin et/ou de métal alcalino-terreux.

L'étape i) permet l'obtention d'un gel précurseur homogène.

Conformément à l'invention, une zéolithe FAU ayant un rapport molaire $SiO_2/Al_2O_3$ compris entre 30 (borne inférieure incluse) et 100 (borne supérieure incluse) et au moins une zéolithe de type structural FAU ayant un ratio molaire $SiO_2/Al_2O_3$ compris entre 2 (borne inférieure incluse) et 30 (borne supérieure exclue), sont incorporées dans le mélange réactionnel pour la mise en oeuvre de l'étape (i) comme sources d'élément silicium et aluminium. Les ratios molaires $SiO_2/Al_2O_3$ des zéolithes FAU incorporés au mélange réactionnel à l'étape i) du procédé selon l'invention sont avantageusement différents entre eux. Avantageusement, le ratio molaire global de l'élément silicium, exprimé sous forme oxyde $SiO_2$, apporté par l'ensemble des zéolithes FAU de départ, par rapport à l'élément aluminium, exprimé sous forme oxyde $Al_2O_3$, apporté par l'ensemble des zéolithes FAU de départ, est compris entre 30 et 80, de préférence entre 32 et 70.

Conformément à l'invention, les zéolithes de type structural FAU mises en oeuvre dans le mélange réactionnel à l'étape i) du procédé selon l'invention ont des ratios molaires $SiO_2/Al_2O_3$ différents et sont telles que : $3250 < P_{ze} < 7200$, de préférence $3350 < P_{ze} < 7100$.

Ainsi, en fonction des zéolithes FAU de départ, dont les ratios molaires $SiO_2/Al_2O_3$ sont différents, et de leurs quantités relatives, le ratio molaire $SiO_2/Al_2O_3$ du gel précurseur du matériau composite AFX-BEA visé peut être ajusté.

Les zéolithes de type structural FAU de départ, celle de ratio molaire $SiO_2/Al_2O_3$ compris entre 30 et 100 et celle(s) de ratio molaire $SiO_2/Al_2O_3$ compris entre 2 et 30 (borne supérieure exclue), peuvent être obtenues par n'importe quelle méthode connue par l'homme du métier, comme par exemple par synthèse directe dans le cas des zéolithes FAU ayant un ratio molaire $SiO_2/Al_2O_3$ inférieur à 6 ou par traitement à la vapeur (steaming) et/ou lavages acides sur une zéolithe de type structural FAU ayant un ratio molaire $SiO_2/Al_2O_3$ inférieur à 6 dans le cas des zéolithes FAU ayant un ratio molaire $SiO_2/Al_2O_3$ supérieur ou égal à 6. Lesdites zéolithes de type structural FAU de départ peuvent être utilisées dans leur forme sodique ou tout autre forme. Elles peuvent par exemple, avant d'être mises en oeuvre dans le procédé selon l'invention, subir un échange d'une partie ou de la totalité de leurs cations sodium avec des cations ammonium, suivi ou non d'une étape de calcination. Parmi les sources de zéolithes FAU de ratio molaire $SiO_2/Al_2O_3$ compris entre 30 et 100 et les sources de zéolithes FAU de ratio molaire $SiO_2/Al_2O_3$ compris entre 2 et 30 (borne supérieure exclue) peuvent être citées les zéolithes commerciales de type Y produites par Zeolyst et par TOSOH, par exemple les zéolithes commerciales CBV100, CBV600, CBV712, CBV720, CBV760 et CBV780, et les zéolithes commerciales HSZ-320HOA, HSZ-350HUA, HSZ-360HUA et HSZ-385HUA.

Conformément à l'invention, l'étape i) de mélange est réalisé en milieu aqueux, c'est-à-dire dans l'eau, de préférence de l'eau déionisée, de telle façon que le ratio molaire $H_2O/(SiO_{2(FAU)})$ est avantageusement compris entre 1 et 100, de préférence entre 5 et 60, $SiO_{2\,(FAU)}$ étant la quantité molaire de $SiO_2$ apportée par la zéolithe FAU de départ, $H_2O$ étant la quantité molaire d'eau présent dans le mélange réactionnel.

Conformément à l'invention, le mélange réactionnel comprend au moins un, de préférence un, composé organique azoté R choisi parmi le dihydroxyde de 1,5-bis(méthylpiperidinium)pentane, le dihydroxyde de 1,6-bis(méthylpiperidinium) hexane le dihydroxyde de 1,7-bis(méthylpiperidinium)heptane et leurs mélanges, ledit composé étant incorporé dans le mélange réactionnel pour la mise en oeuvre de l'étape (i), comme structurant organique. De préférence, le structurant R incorporé dans le mélange réactionnel est le dihydroxyde de 1,6-bis(méthylpiperidinium) hexane. L'anion associé aux cations ammonium quaternaires présents dans l'espèce organique structurante pour la synthèse d'un matériau composite zéolithique AFX-BEA selon l'invention est l'anion hydroxyde.

Le structurant R est incorporé dans le mélange réactionnel de telle façon que le ratio molaire $(R/(SiO_{2\,(FAU)}))$ entre la quantité molaire dudit composé organique azoté R et la quantité molaire de $SiO_2$ apportée par l'ensemble des zéolithes de départ est compris entre 0,01 et 0,6, de préférence entre 0,05 et 0,5.

Conformément à l'invention, au moins une source d'au moins un métal alcalin et/ou alcalino-terreux M de valence n, n étant un nombre entier supérieur ou égal à 1, est mise en oeuvre dans le mélange réactionnel de l'étape i). Le métal alcalin et/ou alcalino-terreux M est de préférence choisi parmi le lithium, le potassium, le sodium, le magnésium et le calcium et le mélange d'au moins deux de ces métaux. De manière très préférée, M est le sodium. De préférence, la source d'au moins un métal alcalin et/ou alcalino-terreux M est l'hydroxyde de sodium.

Avantageusement, la quantité de métal alcalin et/ou alcalino-terreux M de valence n, incorporée dans le mélange réactionnel est telle que le ratio molaire $M_{2/n}O/(SiO_{2\,(FAU)})$ est compris entre 0,005 et 0,45, de préférence entre 0,01 et 0,25, $M_{2/n}O$ étant la quantité molaire de $M_{2/n}O$ apportée par l'ensemble des zéolithes FAU et par la source de métal alcalin et/ou de métal alcalino-terreux, $SiO_{2\ (FAU)}$ la quantité molaire de $SiO_2$ apportée par l'ensemble des zéolithes FAU incorporé dans le mélange réactionnel.

L'étape i) de mélange du procédé selon l'invention est mise en oeuvre jusqu'à obtention d'un mélange réactionnel homogène, appelé gel ou gel précurseur, de préférence pendant une durée supérieure ou égale à 10 minutes et avantageusement pendant moins de 2 heures, en particulier moins de 1,5 heures, de préférence à température ambiante et de préférence sous agitation, à faible ou fort taux de cisaillement, le système d'agitation étant tout système connu de l'homme du métier, par exemple un agitateur mécanique à pâles ou une turbine.

Lors de cet étape i) de mélange, le solvant aqueux introduit peut éventuellement en partie s'évaporer.

L'étape (i) du procédé selon l'invention consiste à préparer un mélange réactionnel aqueux contenant au moins deux zéolithes de type structural FAU, au moins un composé organique azoté R, R étant le dihydroxyde de 1,5-bis(méthylpiperidinium) pentane, le dihydroxyde de 1,6-bis(méthylpiperidinium)hexane et/ou le dihydroxyde de 1,7-bis(méthylpiperidinium)heptane en présence d'au moins une source d'un ou plusieurs métal(aux) alcalin(s) et/ou alcalino-terreux, pour obtenir un gel précurseur d'un matériau composite AFX-BEA. Les quantités desdits réactifs sont ajustées comme indiqué précédemment de manière à conférer à ce gel une composition permettant la cristallisation d'un matériau composite AFX-BEA.

Il peut être avantageux d'ajouter des germes d'une zéolithe de type structural AFX, d'une zéolithe de type structural BEA et/ou d'un matériau composite AFX-BEA au mélange réactionnel au cours de ladite étape i) du procédé de l'invention afin de réduire le temps nécessaire à la formation des cristaux du matériau composite zéolithique et/ou la durée totale de cristallisation. Lesdits germes cristallins favorisent également la formation dudit matériau composite AFX-BEA au détriment d'impuretés. Les germes cristallins sont généralement ajoutés dans une proportion comprise entre 0,01 et 10% de la masse totale des sources desdits élément(s) tétravalent(s) et trivalent(s) considérés sous leurs forme anhydre utilisées dans le mélange réactionnel, lesdits germes cristallins n'étant pas pris en compte dans la masse totale des sources des éléments tétravalents et trivalents. Lesdits germes ne sont pas non plus pris en compte pour déterminer la composition du mélange réactionnel et/ou du gel, définie plus avant, c'est-à-dire dans la détermination des différents rapports molaires de la composition du mélange réactionnel.

Il peut être avantageux de mettre en oeuvre un mûrissement du mélange réactionnel avant la cristallisation hydrothermale au cours de ladite étape i) du procédé de l'invention afin de contrôler la taille des cristaux d'un matériau composite zéolithique AFX-BEA. Ledit mûrissement favorise également la formation dudit matériau composite zéolithique AFX-BEA au détriment d'impuretés. Le mûrissement du mélange réactionnel au cours de ladite étape i) du procédé de l'invention peut être réalisé à température ambiante ou à une température comprise entre 20 et 100°C avec ou sans agitation, pendant une durée avantageusement comprise entre 30 minutes et 48 heures.

**Etape ii) de traitement hydrothermal**

Le procédé de préparation selon l'invention comprend une étape ii) de traitement hydrothermal dudit gel précurseur obtenu à l'issue de l'étape i), éventuellement mûri, à une température comprise entre 120°C et 220°C, de préférence entre 150°C et 195°C, pendant une durée comprise entre 12 heures et 15 jours, de préférence entre 12 heures et 12 jours et de manière plus préférée entre 12 heures et 8 jours. Avantageusement, le traitement hydrothermal est réalisé sous une pression de réaction autogène, éventuellement en ajoutant du gaz, par exemple de l'azote.

Le traitement hydrothermal s'effectue généralement sous agitation ou en absence d'agitation, de préférence sous agitation. Tout système d'agitation connu par l'homme de métier peut être utilisé, par exemple, des pales inclinées avec des contre-pales, des turbines d'agitation, des vis d'Archimède.

Ces conditions opératoires permettent d'obtenir la cristallisation, de préférence complète, du matériau composite visé. La cristallisation est considérée comme complète dès que plus aucune présence de produit amorphe ou de zéolithe FAU n'est détectée par analyse DRX d'un échantillon du milieu réactionnel extrait lors de la synthèse. Selon l'invention, cette étape ii) de traitement hydrothermal peut également être appelée étape de cristallisation, étape de réaction ou encore étape de synthèse. La phase solide cristallisée obtenue est dite « solide ».

A la fin de la réaction, la phase solide formée est avantageusement filtrée, lavée, de préférence à l'eau de préférence déionisée, puis de préférence séchée. Le séchage est généralement réalisé à une température comprise entre 20 et 150°C, de préférence entre 60 et 100°C, pendant une durée comprise entre 5 et 24 heures.

La perte au feu (PAF) dudit solide obtenu après séchage (lorsque ce dernier est effectué en fin d'étape ii)) est généralement comprise entre 4 et 15% poids. Selon l'invention, la perte au feu d'un échantillon, désignée sous l'acronyme PAF, correspond à la différence de masse de l'échantillon testé avant et après un traitement thermique à 1000°C pendant 2 heures. Elle est exprimée en % correspondant au pourcentage de perte de masse. La perte au feu correspond en général à la perte de solvant (comme l'eau) contenu dans le solide mais aussi à l'élimination de composés organiques contenus dans les constituants solides minéraux.

**Etape iii) d'échange**

Le procédé de préparation du catalyseur selon l'invention comprend au moins une étape d'échange ionique comprenant la mise en contact du solide obtenu à l'issue de l'étape précédente, c'est-à-dire du matériau aluminosilicate composite obtenu à l'issue de l'étape ii) ou du matériau aluminosilicate composite séché et calciné obtenu de l'étape iv) dans le cas où les étapes iii) et iv) sont interverties, avec au moins une solution comprenant au moins une espèce apte à libérer un métal de transition, de préférence le cuivre, en solution sous forme réactive, sous agitation, à température ambiante pendant une durée comprise entre 1 heure et 2 jours, avantageusement pendant une durée comprise entre 0,5 jour et 1,5 jours, la concentration en ladite espèce apte à libérer le métal de transition dans ladite solution étant fonction de la quantité de métal de transition que l'on souhaite incorporer audit solide cristallisé ou audit solide cristallisé séché et calciné. De préférence, l'étape iii) d'échange ionique du procédé de préparation selon l'invention est réalisée à l'issue de l'étape ii) de traitement hydrothermal.

Il est également avantageux d'obtenir la forme protonée du matériau aluminosilicate composite après l'étape ii). Ladite forme hydrogène peut être obtenue en effectuant un échange d'ions avec un acide, en particulier un acide minéral fort comme l'acide chlorhydrique, sulfurique ou nitrique, ou avec un composé tel que le chlorure, le sulfate ou le nitrate d'ammonium, avant l'échange ionique avec le ou les métaux de transition.

Le métal de transition libéré dans la solution d'échange est sélectionné dans le groupe formé des éléments suivants : Ti, V, Mn, Mo, Fe, Co, Cu, Cr, Zn, Nb, Ce, Zr, Rh, Pd, Pt, Au, W, Ag. De préférence le métal de transition est Fe, Cu, Nb, Ce ou Mn, préférentiellement Fe ou Cu et de façon encore plus préférée Cu.

Selon l'invention, par « espèce apte à libérer un métal de transition », on entend une espèce apte à se dissocier en milieu aqueux, comme par exemple les sulfates, les nitrates, les chlorures, les oxalates, les complexes organométalliques d'un métal de transition ou leurs mélanges. De préférence, l'espèce apte à libérer un métal de transition est un sulfate ou un nitrate dudit métal de transition.

Selon l'invention, la solution avec laquelle le solide cristallisé ou solide cristallisé séché et calciné est mis en contact, comprend au moins une espèce apte à libérer un métal de transition, de préférence une espèce apte à libérer un métal de transition, de préférence le cuivre.

Avantageusement, le procédé de préparation du catalyseur selon l'invention comprend une étape iii) d'échanges ioniques par mise en contact du solide cristallisé (c'est-à-dire du solide obtenu à l'issue de l'étape ii) ou cristallisé, séché et calciné (c'est-à-dire du solide obtenu à l'issue de l'étape iv dans le cas où les étapes iii) et iv) sont interverties), avec une solution comprenant au moins une espèce, de préférence une seule espèce, apte à libérer un métal de transition ou par mise en contact successive dudit solide avec plusieurs solutions comprenant chacune au moins une espèce, de préférence une espèce, apte à libérer un métal de transition, les différentes solutions comprenant avantageusement des espèces aptes à libérer un métal de transition différentes.

Selon l'invention, la solution avec laquelle le solide cristallisé est mis en contact comprend au moins une espèce apte à libérer un métal de transition, de préférence une seule espèce apte à libérer un métal de transition, de préférence le fer ou cuivre, préférentiellement le cuivre.

A la fin de l'échange, le solide obtenu est avantageusement filtré, lavé, par exemple à l'eau déionisée, et, de préférence, ensuite séché pour obtenir une poudre. Dans le cas où l'étape iii) comprend des mises en contact successives du solide avec différentes solutions, le solide obtenu après chaque mise en contact peut être avantageusement filtré, lavé, par exemple à l'eau déionisée, puis, de préférence, séché. Le séchage est généralement réalisé à une température comprise entre 20 et 150°C, de préférence entre 60 et 100°C, pendant une durée comprise entre 5 et 24 heures.

La quantité totale de métal de transition, de préférence le cuivre, contenue dans ledit catalyseur final est comprise entre 0,5 et 6% massique, de préférence entre 0,5 et 5% massique, et de façon encore plus préférée entre 1 et 4% massique, par rapport à la masse totale du catalyseur sous sa forme anhydre.

Selon un mode de réalisation de l'invention dans lequel le catalyseur ne contient que du cuivre comme métal de transition, la teneur massique en cuivre incorporé au catalyseur se situe entre 0,5 et 6%, de préférence entre 0,5 et 5%, préférablement entre 1 et 4% et de manière encore plus préférée entre 1,5 et 3,5% par rapport à la masse totale du catalyseur final sous sa forme anhydre.

Selon un autre mode de réalisation de l'invention dans lequel le catalyseur comprend du cuivre et un autre métal de transition, de préférence, Fe, Nb, Ce, Mn, la teneur en cuivre du catalyseur obtenu se situe entre 0,05 et 2% massique, de préférence 0,5 et 2% massique alors que celle de l'autre métal de transition se situe préférablement entre 1 et 4% massique, les teneurs en métaux de transition étant données en pourcentage massiques par rapport à la masse totale du catalyseur final sous sa forme anhydre. Dans ce mode de réalisation, l'étape iii) d'échange est de préférence mise en oeuvre par mise en contact successive de deux solutions contenant pour l'une une espèce apte à libérer le cuivre en solution et pour l'autre solution une espèce apte à libérer un métal de transition autre que le cuivre, de préférence Fe, Nb, Ce, Mn.

Selon un troisième mode de réalisation de l'invention dans lequel le catalyseur ne contient que le fer comme métal de transition, la teneur massique en fer se situe entre 0,5 et 5%, de préférence entre 1 et 4% et préférablement entre 1,5 et 3,5% par rapport à la masse totale du catalyseur final sous sa forme anhydre.

Selon un autre mode particulier de réalisation de l'invention dans lequel le catalyseur comprend du fer et un autre métal de transition, de préférence Cu, Nb, Ce, Mn, la teneur en fer du catalyseur obtenu se situe entre 0,05 et 2% massique, de préférence entre 0,5 et 2% massique alors que celle de l'autre métal de transition se situe préférablement entre 1 et 4% massique, les teneurs en métaux de transition étant données en pourcentage massiques par rapport à la masse totale du catalyseur final sous sa forme anhydre. Dans ce mode de réalisation, l'étape iii) d'échange est avantageusement mise en oeuvre par mise en contact successive de deux solutions contenant pour l'une une espèce apte à libérer le fer en solution et pour l'autre solution une espèce apte à libérer un métal de transition autre que le cuivre, de préférence Cu, Nb, Ce, Mn.

### Etape iv) de traitement thermique

Le procédé de préparation selon l'invention comprend une étape iv) de traitement thermique réalisée à l'issue de l'étape précédente, c'est-à-dire à l'issue de l'étape ii) de traitement hydrothermal ou à l'issue de l'étape iii) d'échange, de préférence à l'issue de l'étape iii) d'échange ionique. Selon l'invention, les deux étapes iii) et iv) peuvent avantageusement être interverties et également éventuellement être répétées. Chacune des deux étapes iii) et iv) peut aussi éventuellement être répétée individuellement.

Ladite étape iv) de traitement thermique comprend un séchage du solide à une température comprise entre 20 et 150°C, de préférence entre 60 et 100°C, pendant une durée compris entre 2 et 24 heures, suivi d'une calcination, correspondant à un traitement par combustion sous air, éventuellement sec, à une température comprise entre 450 et 700°C, de préférence entre 500 et 600°C pendant une durée comprise entre 2 et 20 heures, de préférence entre 5 et 10 heures, préférentiellement entre 6 et 9 heures, le débit d'air éventuellement sec étant de manière préférée compris entre 0,5 et 1,5 L/h/g de solide à traiter, préférentiellement compris entre 0,7 et 1,2 L/h/g de solide à traiter. La calcination peut être précédée d'une montée en température progressive.

En particulier, le catalyseur obtenu par un procédé comprenant au moins les étapes i), ii), iii), et iv) précédemment décrites présente des propriétés améliorées pour la conversion des $NO_x$.

### Caractérisation du catalyseur préparé selon l'invention

Le catalyseur selon l'invention comprend une structure zéolithique composé d'un mélange de zéolithes de structure AFX et de structure BEA selon la classification de l'International Zeolite Association (IZA), échangé par au moins un métal de transition. Cette structure est caractérisée par diffraction aux rayons X (DRX).

La technique de diffraction des rayons X permet également de déterminer la proportion massique du mélange des zéolithes AFX et BEA dans ledit catalyseur et les proportions relatives de chaque zéolithe, AFX et BEA. Avantageusement, la proportion massique globale des zéolithes AFX et BEA dans le catalyseur obtenu est supérieure ou égale à 90%, de préférence supérieure ou égale à 95%, préférentiellement supérieure ou égale à 99% et de manière encore plus préférée supérieure ou égale à 99,8%, par rapport à la masse totale dudit catalyseur, sous sa forme anhydre. En d'autres termes, le catalyseur obtenu comprend moins de 10% massique, de préférence moins de 5% massique, préférentiellement moins de 1% massique et de manière encore plus préférée moins de 0,2% massique d'impuretés et/ou de phase cristalline ou amorphe autre que AFX et BEA (les bornes n'étant pas incluses). De manière très avantageuse, le procédé de l'invention conduit à la formation d'un catalyseur comprenant une structure zéolithique composée d'un mélange de zéolithes AFX et BEA, exempte de toute autre phase cristalline ou amorphe et/ou toute impureté.

Le diagramme de diffraction aux rayons X (DRX) est obtenu par analyse radiocristallographique au moyen d'un diffractomètre en utilisant la méthode classique des poudres avec le rayonnement $K\alpha_1$ du cuivre ($\lambda$ = 1,5406Å). A partir de la position des pics de diffraction représentée par l'angle 2θ, on calcule, par la relation de Bragg, les équidistances réticulaires $d_{hkl}$ caractéristiques de l'échantillon. L'erreur de mesure $\Delta(d_{hkl})$ sur $d_{hkl}$ est calculée grâce à la relation de Bragg en fonction de l'erreur absolue $\Delta(2\theta)$ affectée à la mesure de 2θ. Une erreur absolue $\Delta(2\theta)$ égale à ± 0,02° est communément admise. L'intensité relative $I_{rel}$ affectée à chaque valeur de $d_{hkl}$ est mesurée d'après la hauteur du pic de diffraction correspondant. La comparaison du diffractogramme avec les fiches de la base de données d'ICDD (International Centre for Diffraction Data) en utilisant un logiciel comme par exemple le DIFFRACT.SUITE nous permet aussi de faire l'identification des phases cristallines présentes dans le matériau obtenu.

Le diagramme de diffraction des rayons X du matériau composite AFX-BEA obtenu à l'issue de l'étape ii) du procédé selon l'invention ou du catalyseur selon l'invention, avantageusement obtenu à l'issue de l'étape iv) du procédé de l'invention, comporte au moins les raies aux valeurs de dhkl données dans le Tableau 1. Dans la colonne des $d_{hkl}$, les valeurs moyennes des distances inter-réticulaires en Angströms (Å) sont indiquées. Chacune de ces valeurs doit être affectée de l'erreur de mesure Δ(dhkl) comprise entre ± 0,6Å et ± 0,01Å.

Tableau 1 : Valeurs moyennes des $d_{hkl}$ et intensités relatives mesurées sur un diagramme de diffraction de rayons X du catalyseur selon l'invention ou du matériau composite obtenu à l'issue de l'étape ii) du procédé selon l'invention

| 2 thêta (°) | dhkl (Å) | Irel | 2 thêta (°) | dhkl (Å) | Irel |
|---|---|---|---|---|---|
| 7,49 | 11,79 | ff-f | 26,11 | 3,41 | f-m |
| 7,71 | 11,46 | ff-f | 26,94 | 3,31 | ff |
| 8,71 | 10,14 | mf-m | 27,11 | 3,29 | ff |
| 11,66 | 7,59 | f-mf | 27,61 | 3,23 | ff |
| 12,97 | 6,82 | f | 28,04 | 3,18 | mf-m |
| 15,00 | 5,90 | ff | 28,68 | 3,11 | ff |
| 15,40 | 5,75 | ff | 29,51 | 3,03 | ff |
| 15,66 | 5,66 | f-mf | 30,19 | 2,96 | ff-f |
| 17,47 | 5,07 | mf | 30,58 | 2,92 | mf |
| 17,90 | 4,95 | f-mf | 30,99 | 2,88 | ff |
| 19,42 | 4,57 | ff | 31,59 | 2,83 | f-mf |
| 19,88 | 4,46 | ff-f | 32,50 | 2,75 | ff |
| 20,38 | 4,36 | F-FF | 33,73 | 2,66 | f-mf |
| 21,08 | 4,21 | f | 34,29 | 2,61 | ff |
| 21,31 | 4,17 | ff-f | 34,78 | 2,58 | ff |
| 21,82 | 4,07 | F-FF | 35,11 | 2,55 | ff |
| 22,19 | 4,00 | f-m | 35,79 | 2,51 | ff |
| 22,34 | 3,98 | f-FF | 37,56 | 2,39 | ff |
| 22,54 | 3,94 | ff-f | 38,00 | 2,37 | ff |
| 22,70 | 3,91 | ff-f | 39,18 | 2,30 | ff |
| 23,67 | 3,76 | mf | 39,61 | 2,30 | ff |
| 25,24 | 3,52 | ff | | | |

où FF = très fort ; F = fort ; m = moyen ; mf = moyen faible ; f = faible ; ff = très faible. L'intensité relative $I_{rel}$ est donnée en rapport à une échelle d'intensité relative où il est attribué une valeur de 100 à la raie la plus intense du diagramme de diffraction des rayons X : ff < 15 ; 15 ≤ f < 30 ; 30 ≤ mf < 50 ; 50 ≤ m < 65 ; 65 ≤ F < 85 ; FF ≥ 85 ; 1 ≤ ff-f < 30 ; 30 ≤ mf-m < 65 ; 15 ≤ f-mf < 50 ; 65 ≤ F-FF ≤ 100 ; 15 ≤ f-m < 65 ; 15 ≤ f-FF ≤ 100.

Selon l'invention, la composition massique du catalyseur, en particulier les fractions massiques relatives des zéolithes de type structural AFX et de type structural BEA présentes dans ledit catalyseur, est avantageusement déterminée à l'aide d'une méthode semblable à la norme ASTM D3906 03, par comparaison des surfaces des pics aux angles (2θ) 20,38 ± 0,1 (hkl : 211) ; 23,67 ± 0,1 (hkl : 105) ; 26,1 ± 0,1 (hkl : 303) et 28,02 ± 0,1 (hkl : 106) des diagrammes des rayons X obtenus pour le matériau composite selon l'invention et une zéolithe de type structural AFX de référence, de préférence de haute pureté, avantageusement ayant une pureté supérieure ou égale à 99,8%. Le rapport massique des deux zéolithes AFX et BEA dans le matériau composite selon l'invention est ainsi évalué en comparant la somme des surface des pics aux angles (2θ) cités ci-avant obtenue pour le matériau composite préparé selon l'invention avec celle obtenue pour un échantillon de référence de zéolithe AFX et en utilisant la formule de calcul suivante :

$$AFX/BEA = SAFXc/(SAFXr - SAFXc)$$

où SAFXc est la somme des surfaces des pics présents aux angles (2□) 20,38 ± 0,1 (hkl : 211) ; 23,67 ± 0,1 (hkl : 105) ; 26,1 ± 0,1 (hkl : 303) et 28,02 ± 0,1 (hkl : 106) du diffractogramme du matériau composite AFX-BEA contenant du cuivre préparé selon l'invention et SAFXr est la somme des surfaces des pics présents aux angles (2□) : 20,38 (hkl :

211) ; 23,67 (hkl : 105) ; 26,1 (hkl : 303) et 28,02 (hkl : 106) du diffractogramme de la zéolithe de type structural AFX pure, utilisée comme référence.

L'analyse qualitative et quantitative des espèces chimiques présentes dans les matériaux obtenus est faite par spectrométrie de fluorescence des rayons X (FX). Celle-ci est une technique d'analyse chimique utilisant une propriété physique de la matière, la fluorescence de rayons X. Le spectre des rayons X émis par la matière est caractéristique de la composition de l'échantillon, en analysant ce spectre, on peut en déduire la composition élémentaire, c'est-à-dire les concentrations massiques en éléments.

La perte au feu (PAF) du catalyseur obtenu après l'étape de séchage (et avant calcination) ou après l'étape de calcination de l'étape iv) du procédé selon l'invention est généralement comprise entre 2 et 15% poids, de préférence entre 4 et 15% poids. La perte au feu d'un échantillon de catalyseur, désignée sous l'acronyme PAF, correspond à la différence de masse de l'échantillon avant et après un traitement thermique à 1000°C pendant 2 heures. Elle est exprimée en % correspondant au pourcentage de perte de masse. La perte au feu correspond en général à la perte de solvant (comme l'eau) contenu dans le solide mais aussi à l'élimination de composés organiques contenus dans les constituants solides minéraux.

## Utilisation du catalyseur selon l'invention

L'invention concerne également l'utilisation du catalyseur selon l'invention, avantageusement directement préparé ou susceptible d'être préparé par le procédé décrit précédemment, pour la réduction sélective de $NO_x$ par un réducteur tel que $NH_3$ ou $H_2$, avantageusement mis en forme par dépôt sous forme de revêtement (« washcoat » selon la terminologie anglo-saxonne) sur un monolithe (ou structure), de préférence une structure nid d'abeilles principalement pour les applications mobiles ou une structure à plaques que l'on retrouve particulièrement pour les applications stationnaires.

La structure nid d'abeilles est formée de canaux parallèles ouverts aux deux extrémités (flow-through en anglais) ou comporte des parois poreuses filtrantes et dans ce cas les canaux parallèles adjacents sont alternativement bouchés de part et d'autre des canaux afin de forcer le flux de gaz à traverser la paroi (wall-flow monolith en anglais). Ladite structure nid d'abeilles ainsi revêtue constitue un pain catalytique. Ladite structure peut être composée de cordiérite, carbure de silicium (SiC), titanate d'aluminium (AlTi), alumine alpha, mullite ou tout autre matériau dont la porosité est comprise entre 30 et 70%. Ladite structure peut être réalisée en tôle métallique, en acier inoxydable contenant du chrome et de l'aluminium, acier de type FeCrAl.

La quantité de catalyseur selon l'invention déposé sur ladite structure nid d'abeille est comprise entre 50 à 180 g/L pour les structures filtrantes et entre 80 et 200 g/L pour les structures avec canaux ouverts.

Le catalyseur selon l'invention est avantageusement associé à un liant tel que la cérine, l'oxyde de zirconium, l'alumine, la silice-alumine non zéolithique, l'oxyde de titane, un oxyde mixte de type cerine-zircone, un oxyde de tungstène, une spinelle, pour être mis en forme par dépôt sous forme de revêtement. Ledit revêtement est avantageusement appliqué à une structure monolithique, ou monolithe, de manière très préférée à une structure nid d'abeilles, par une méthode de dépôt (« washcoating » en anglais) qui consiste à tremper le monolithe dans une suspension (slurry en anglais) de poudre de catalyseur selon l'invention dans un solvant, de préférence de l'eau, et potentiellement des liants, oxydes métalliques, stabilisateurs ou autres promoteurs. Cette étape de trempe peut être répétée jusqu'à atteindre la quantité souhaitée de revêtement. Dans certains cas le slurry peut aussi être pulvérisé au sein du monolithe. Le revêtement une fois déposé, le monolithe est calciné à une température de 300 à 600°C pendant 1 à 10 heures.

Ladite structure monolithique peut être revêtue d'une ou plusieurs revêtements. Le revêtement comprenant le catalyseur selon l'invention est avantageusement associé à, c'est-à-dire recouvre un ou est recouvert par, un autre revêtement présentant des capacités d'adsorption de polluants en particulier de NOx, de réduction de polluants en particulier des NOx ou favorisant l'oxydation de polluants, en particulier celle de l'ammoniac.

Une autre possibilité est de mettre le catalyseur sous forme d'extrudé. Dans ce cas, la structure obtenue peut contenir jusqu'à 100% de catalyseur selon l'invention.

Ladite structure revêtue par le catalyseur selon l'invention ou obtenue par extrusion du catalyseur selon l'invention est avantageusement intégrée dans une ligne d'échappement d'un moteur à combustion interne fonctionnant principalement en mélange pauvre, c'est-à-dire en excès d'air par rapport à la stoechiométrie de la réaction de combustion comme c'est le cas pour les moteurs Diesel par exemple. Dans ces conditions de fonctionnement du moteur, les gaz d'échappement contiennent notamment les polluants suivants : des suies, des hydrocarbures imbrulés (HC), du monoxyde de carbone (CO), des oxydes d'azotes (NOx). En amont de ladite structure revêtue du catalyseur selon l'invention peut être placé un catalyseur d'oxydation dont la fonction est d'oxyder les HC et le CO ainsi qu'un filtre pour éliminer les suies des gaz d'échappement, la fonction de ladite structure revêtue étant d'éliminer le NOx, sa gamme de fonctionnement de se situant entre 100 et 900°C et de manière préférée entre 200°C et 500°C.

## LISTE DES FIGURES

La Figure 1 représente les formules chimiques des composés organiques azotés qui peuvent être choisis comme structurant utilisé dans le procédé de synthèse selon l'invention.

La Figure 2 représente le diagramme de diffraction X du catalyseur Cu-AFX-BEA obtenu selon l'exemple 2.

L'invention est illustrée par les exemples suivants qui ne présentent, en aucun cas, un caractère limitatif.

## EXEMPLES

**Exemple 1** : *préparation du dihydroxyde de 1,6-bis(méthylpiperidinium)hexane (structurant R).*

50 g de 1,6-dibromohexane (0,20 mole, 99%, Alfa Aesar) sont ajoutés dans un ballon de 1 L contenant 50 g de N-méthylpipéridine (0,51 mole, 99%, Alfa Aesar) et 200 mL d'éthanol. Le milieu réactionnel est agité et porté à reflux pendant 5 heures. Le mélange est ensuite refroidi à température ambiante puis filtré. Le mélange est versé dans 300 mL de diéthyléther froid puis le précipité formé est filtré et lavé avec 100 mL de diéthyléther. Le solide obtenu est recristallisé dans un mélange éthanol/éther. Le solide obtenu est séché sous vide pendant 12 heures. On obtient 71 g d'un solide blanc (soit un rendement de 80 %).
Le produit possède le spectre RMN 1H attendu. RMN 1H (D2O, ppm/TMS) : 1,27 (4H,m) ; 1,48 (4H,m) ; 1,61 (4H,m) ; 1,70 (8H,m) ; 2,85 (6H,s) ; 3,16 (12H,m). Ce spectre RMN 1H correspond à celui du dibromure de 1,6-bis(méthylpiperidinium)hexane.
18,9 g d'Ag2O (0,08 mole, 99%, Aldrich) sont ajoutés dans un bécher en téflon de 250 ml contenant 30 g du dibromure de 1,6-bis(méthylpiperidinium)hexane (0,07 mole) préparé et 100 ml d'eau déionisée. Le milieu réactionnel est agité à l'abri de la lumière pendant 12 heures. Le mélange est ensuite filtré. Le filtrat obtenu est composé d'une solution aqueuse de dihydroxyde de 1,6-bis(méthylpiperidinium)hexane. Le dosage de cette espèce est réalisé par RMN du proton en utilisant l'acide formique en tant qu'étalon.

**Exemple 2** : *préparation d'un catalyseur selon l'invention à 2% Cu*

0,239 g d'une zéolithe de type structural FAU (CBV712 Zeolyst, $SiO_2/Al_2O_3$= 11,42, PAF = 12,81) ont été mélangés avec 4,952 g d'eau déionisée. 0,573 g d'une zéolithe de type structural FAU (CBV780 Zeolyst, $SiO_2/Al_2O_3$= 98,22, PAF = 8,52, Pze = 7170) sont ajoutés au mélange précédent, la préparation obtenue est maintenue sous agitation pendant 10 minutes. 2,905 g d'une solution aqueuse de dihydroxyde de 1,6-bis(méthylpiperidinium)hexane (20,91 % en poids) préparé selon l'exemple 1, sont ajoutés au mélange précédent. Le mélange est alors maintenu sous agitation pendant 10 minutes. 0,330 g d'une solution aqueuse à 20% en poids d'hydroxyde de sodium (solution préparée à partir d'hydroxyde de sodium à 98% en poids, Aldrich) sont ajoutés dans le mélange et maintenu sous agitation pendant 10 minutes. La composition molaire du gel précurseur est la suivante: 60 $SiO_2$: 1,8 $Al_2O_3$: 10 $R(OH)_2$: 4,3 $Na_2O$: 2204 $H_2O$, soit un ratio $SiO_2/Al_2O_3$ de 33,3.
Le gel précurseur est ensuite transféré, après homogénéisation dans un autoclave. L'autoclave est fermé puis chauffé pendant 6 jours à 180°C sous agitation à 35 tr/min avec un système tourne-broche. Le produit cristallisé obtenu est filtré, lavé à l'eau déionisée.

*Etape de traitement thermique (calcination)*

Le solide lavé est séché une nuit à 100°C. La perte au feu (PAF) du solide séché, évaluée à 1000°C pendant 2 heures, est de 10,1%.
Le solide séché est ensuite introduit dans un four à moufle où est réalisée une étape de calcination sous flux d'air: le cycle de calcination comprend une montée de 1,5°C/min en température jusqu'à 200°C, un palier à 200°C maintenu durant 2 heures, une montée de 1°C/min en température jusqu'à 550°C suivi d'un palier à 550°C maintenu durant 8 heures puis un retour à la température ambiante.
Le produit solide calciné a été analysé par diffraction des rayons X et identifié comme étant constitué par un mélange d'environ 50% massique d'une zéolithe de type structural AFX et 50% massique d'une zéolithe de type structural BEA. Le mélange AFX-BEA représente environ 100% massique du produit obtenu.

*Echange ionique au NH$_4$$^+$ sur la zéolithe AFX calcinée et Traitement thermique*

Le matériau composite AFX-BEA calciné est mis en contact avec une solution de NH$_4$NO$_3$ 3 molaire pendant 1 heure sous agitation à 80°C. Le rapport entre le volume de solution de NH$_4$NO$_3$ et la masse de solide est de 10. Le solide obtenu est filtré et lavé et la procédure d'échange est répété encore deux fois dans les mêmes conditions. Le solide final est séparé, lavé à l'eau déionisée et séché à 100°C pendant 4 heures.

Le matériau composite AFX-BEA sous forme ammoniacale est traité sous flux d'air à 550°C pendant 8 heures avec une rampe de montée en température de 1°C/min. La perte au feu (PAF) du solide obtenu, évaluée à 1000°C pendant 2 heures, est de 4% poids. Le produit obtenu est un matériau composite AFX-BEA sous forme protonée.

*Echange ionique au Cu et Traitement thermique*

Le matériau composite AFX-BEA calciné est mis en contact avec une solution de [Cu(NH$_3$)$_4$](NO$_3$)$_2$ pendant 1 journée sous agitation à température ambiante. Le solide final est séparé, lavé à l'eau déionisée et séché à 100°C pendant 4 heures.

Le solide échangé et séché, obtenu après la mise en contact avec la solution de [Cu(NH$_3$)$_4$](NO$_3$)$_2$ est calciné sous flux d'air à 550°C durant 8 heures.

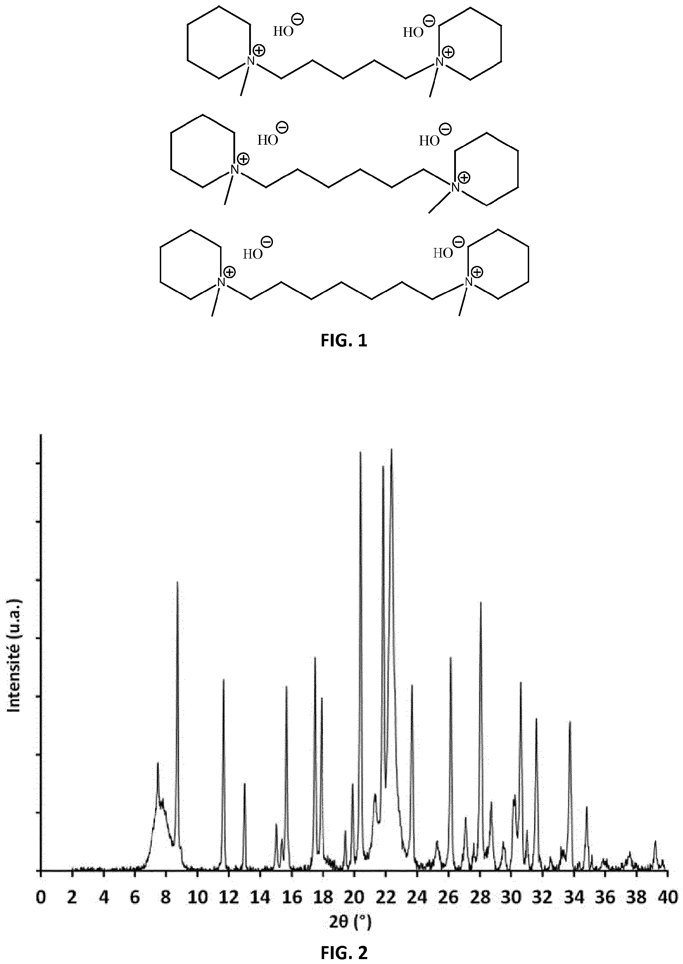

Le produit solide calciné est analysé par diffraction des rayons X (cf. Figure 2) et identifié comme étant constitué par un mélange d'environ 50% massique d'une zéolithe de type structural AFX et 50% massique d'une zéolithe de type structural BEA. Le mélange AFX-BEA représente 98% massique du produit obtenu. La teneur en cuivre représente un pourcentage massique de 2% tel que déterminé par fluorescence X.

Le catalyseur obtenu est noté Cu-AFX-BEA.

**Exemple 3 :** *Conversion des NOx en Standard SCR*

Un test catalytique de réduction des oxydes d'azote (NOx) par l'ammoniac (NH$_3$) en présence d'oxygène (O$_2$) dans des conditions Standard SCR est réalisé à différentes températures de fonctionnement pour le catalyseur synthétisé suivant l'exemple 2 (Cu-AFX-BEA).

200 mg de catalyseur sous forme de poudre est disposé dans un réacteur en quartz. 145 l/h d'une charge représentative d'un mélange de gaz d'échappement d'un moteur Diesel sont alimentés dans le réacteur. Cette charge présente la composition molaire suivante : 400 ppm NO, 400 ppm NH$_3$, 8,5% O$_2$, 9% CO$_2$, 10% H$_2$O, qpc N$_2$.

Un analyseur FTIR permet de mesurer la concentration des espèces NO, NO$_2$, NH$_3$, N$_2$O, CO, CO$_2$, H$_2$O, O$_2$ en sortie de réacteur. Les conversions de NOx calculées comme suit :

$$\text{Conversion} = (\text{NOx entrée} - \text{NOx sortie}) / \text{NOx entrée}$$

Les résultats, en particulier les températures d'amorçage du catalyseur, sont donnés ci-dessous pour les conditions Standard-SCR :

|  | T50 | T80 | T90 | T100 |
|---|---|---|---|---|
| Cu-AFX-BEA | 196°C | 238°C | 280°C | 400°C |

T50 correspond à la température à laquelle 50% des NOx du mélange gazeux sont convertis par le catalyseur. T80 correspond à la température à laquelle 80% des NOx du mélange gazeux sont convertis par le catalyseur. T90 correspond à la température à laquelle 90% des NOx du mélange gazeux sont convertis par le catalyseur. T100 correspond à la température à laquelle 100% des NOx du mélange gazeux sont convertis par le catalyseur.

Il apparait que le catalyseur Cu-AFX-BEA synthétisé selon l'invention permet de convertir efficacement les NOx sur l'ensemble de la plage de température testée. Une conversion maximum de 100% est atteinte à 400°C. Les températures d'amorçage obtenues avec le catalyseur Cu-AFX-BEA selon l'invention sont satisfaisantes : elles sont en effet faibles, notamment pour les taux de conversion 50%, 80% et 90%.

**Revendications**

**1.** Procédé de préparation d'un catalyseur comprenant un mélange de zéolithes de type structural AFX et de type structural BEA, et au moins un métal de transition, comprenant au moins les étapes suivantes :

i) le mélange en milieu aqueux, d'une zéolithe FAU ayant un rapport molaire $SiO_2/Al_2O_3$ compris entre 30 et 100 avec au moins une zéolithe de type structural FAU ayant un ratio molaire $SiO_2/Al_2O_3$ compris entre 2 et 30 (borne supérieure exclue), et où le paramètre mathématique, $P_{ze}$, correspondant au pourcentage massique de la zéolithe FAU de rapport molaire $SiO_2/Al_2O_3$ compris entre 30 et 100, sous sa forme anhydre (exprimé en %) dans le mélange de zéolithes FAU, multiplié par le rapport molaire $SiO_2/Al_2O_3$ de ladite même zéolithe FAU de rapport molaire $SiO_2/Al_2O_3$ compris entre 30 et 100, est tel que : $3250 < P_{ze} < 7200$, d'au moins un composé organique azoté R, choisi parmi le dihydroxyde de 1,5-bis(méthylpiperidinium)pentane, le dihydroxyde de 1,6-bis(méthylpiperidinium)hexane, le dihydroxyde de 1,7-bis(méthylpiperidinium) heptane et leurs mélanges, au moins une source d'au moins un métal alcalin et/ou métal alcalino-terreux M de valence n, n étant un nombre entier supérieur ou égal à 1, le mélange réactionnel présentant la composition molaire suivante :

| | |
|---|---|
| $(SiO_{2(FAU)})/(Al_2O_{3(FAU)})$ | compris entre 30 et 80, |
| $H_2O/(SiO_{2(FAU)})$ | compris entre 1 et 100, |
| $R/(SiO_{2(FAU)})$ | compris entre 0,01 et 0,6, |
| $M_{2/n}O/(SiO_{2(FAU)})$ | compris entre 0,005 et 0,45, |

dans laquelle $SiO_{2\ (FAU)}$ est la quantité molaire de $SiO_2$ apportée par l'ensemble des zéolithes de type structural FAU introduites dans le mélange, $Al_2O_{3\ (FAU)}$ est la quantité molaire de $Al_2O_3$ apportée par l'ensemble des zéolithes de type structural FAU introduites dans le mélange, $H_2O$ la quantité molaire d'eau présente dans le mélange réactionnel, R la quantité molaire dudit composé organique azoté, $M_{2/n}O$ étant la quantité molaire de $M_{2/n}O$ apportée par l'ensemble des zéolithes FAU et par la source de métal alcalin et/ou de métal alcalino-terreux, jusqu'à l'obtention d'un gel précurseur;

ii) le traitement hydrothermal dudit gel précurseur obtenu à l'issue de l'étape i) à une température comprise entre 120°C et 220°C, pendant une durée comprise entre 12 heures et 15 jours, pour obtenir une phase solide cristallisée, dite « solide » ;

iii) au moins un échange ionique comprenant la mise en contact du solide obtenu à l'issue de l'étape précédente, avec au moins une solution comprenant au moins une espèce apte à libérer un métal de transition en solution sous forme réactive sous agitation à température ambiante pendant une durée comprise entre 1 heure et 2 jours, le métal de transition libéré dans la solution de l'étape iii) étant sélectionné dans le groupe formé des éléments suivants : Ti, V, Mn, Mo, Fe, Co, Cu, Cr, Zn, Nb, Ce, Zr, Rh, Pd, Pt, Au, W, Ag, et la teneur en métal de transition étant comprise entre 0,5 à 6% massique, par rapport à la masse totale du catalyseur final, sous sa forme anhydre ;

iv) le traitement thermique par séchage du solide obtenu à l'issue de l'étape précédente à une température comprise entre 20 et 150°C pendant une durée comprise entre 2 et 24 heures, suivi d'une calcination sous flux de d'air à une température comprise entre 450 et 700°C pendant une durée comprise entre 2 et 20 heures.

2. Procédé selon la revendication 1, dans lequel les étapes iii) et iv) sont interverties, et éventuellement répétées.

3. Procédé selon la revendication 1 ou 2, dans lequel l'étape iii) est réalisée par mise en contact du solide avec une solution comprenant au moins une espèce, de préférence une seule espèce, apte à libérer un métal de transition ou par mise en contact successive du solide avec différentes solutions comprenant chacune au moins une, de préférence une seule, espèce apte à libérer un métal de transition, les différentes solutions comprenant avantageusement des espèces aptes à libérer un métal de transition différentes.

4. Procédé selon l'une des revendications 1 à 3, dans lequel le métal de transition libéré dans la solution de l'étape iii) est Fe, Cu, Nb, Ce ou Mn, , préférentiellement Fe ou Cu et de façon encore plus préférée Cu.

5. Procédé selon l'une des revendications 1 à 4, dans lequel la teneur en métal de transition est comprise entre 0,5 et 5% massique, et de façon encore plus préférée entre 1 et 4% massique, par rapport à la masse totale du catalyseur final, sous sa forme anhydre, la quantité de cuivre du matériau composite dudit catalyseur étant prise en compte.

6. Procédé selon l'une des revendications 1 à 5, dans lequel le catalyseur ne contient que du cuivre comme métal de transition, à une teneur massique comprise entre 0,5 et 6%, de préférence entre 0,5 et 5%, préférablement entre 1 et 4% et de manière plus préférée encore entre 1,5 et 3,5% par rapport à la masse totale du catalyseur final sous sa forme anhydre.

**7.** Procédé selon l'une des revendications 1 à 5, dans lequel le catalyseur comprend du cuivre et un autre métal de transition, de préférence, Fe, Nb, Ce, Mn, la teneur en cuivre du catalyseur obtenu se situe entre 0,05 et 2% massique, de préférence 0,5 et 2% massique alors que celle de l'autre métal de transition se situe préférablement entre 1 et 4% massique, les teneurs en métaux de transition étant données en pourcentage massiques par rapport à la masse totale du catalyseur final sous sa forme anhydre.

**8.** Procédé selon l'une des revendications 1 à 5, dans lequel le catalyseur ne contient que du fer comme métal de transition, à une teneur massique comprise entre 0,5 et 5%, préférablement entre 1 et 4% et de manière plus préférée encore entre 1,5 et 3,5% par rapport à la masse totale du catalyseur final sous sa forme anhydre.

**9.** Procédé selon l'une des revendications 1 à 5, dans lequel le catalyseur comprend du fer et un autre métal de transition, de préférence Cu, Nb, Ce, Mn, la teneur en fer du catalyseur obtenu se situe entre 0,05 et 2% massique, de préférence entre 0,5 et 2% massique alors que celle de l'autre métal de transition se situe préférablement entre 1 et 4% massique, les teneurs en métaux de transition étant données en pourcentage massiques par rapport à la masse totale du catalyseur final sous sa forme anhydre.

**10.** Procédé selon l'une des revendications précédentes, pour lequel le composé organique azoté R est le dihydroxyde de 1,6-bis(méthylpiperidinium)hexane.

**11.** Procédé selon l'une des revendications précédentes, dans lequel le traitement hydrothermal de l'étape ii) est réalisé à une température comprise entre 120°C et 220°C, de préférence entre 150°C et 195°C, pendant une durée comprise entre 12 heures et 15 jours, de préférence entre 12 heures et 12 jours, et de manière plus préférée entre 12 heures et 8 jours, et de préférence sous pression autogène.

**12.** Procédé selon l'une des revendications précédentes, pour lequel l'étape iv) de traitement thermique comprend un séchage du solide à une température comprise entre 20 et 150°C, de préférence entre 60 et 100°C, pendant une durée compris entre 2 et 24 heures, suivi d'une calcination, correspondant à un traitement par combustion sous air éventuellement sec, à une température comprise entre 450 et 700°C, préférentiellement entre 500 et 600°C pendant une durée comprise entre 5 et 10 heures, préférentiellement entre 6 et 9 heures, le débit d'air éventuellement sec étant de manière préférée compris entre 0,5 et 1,5 L/h/g, préférentiellement compris entre 0,7 et 1,2 L/h/g de solide à traiter.

**13.** Catalyseur comprenant un matériau composite contenant un mélange intime d'une zéolithe de type AFX et d'une zéolithe de type BEA, et au moins un métal de transition, présentant une structure zéolithique comprenant :

- entre 30 et 90% massique, de préférence entre 40 et 90% massique de zéolithe de type structural AFX, par rapport à la masse totale dudit catalyseur sous sa forme anhydre ;
- entre 10 et 70% massique, de préférence entre 10 et 60% massique de zéolithe de type structural BEA, par rapport à la masse totale dudit catalyseur sous sa forme anhydre ;
le catalyseur présentant un rapport molaire $SiO_2/Al_2O_3$ compris entre 2 et 100, de préférence compris entre 4 et 90 et plus préférentiellement entre 6 et 80,
dans lequel ledit métal de transition est sélectionné dans le groupe formé des éléments suivants : Ti, V, Mn, Mo, Fe, Co, Cu, Cr, Zn, Nb, Ce, Zr, Rh, Pd, Pt, Au, W, Ag, de préférence Cu, Fe, Nb, Ce, Mn, de manière très préférée le fer, le cuivre ou leur mélange,
dans lequel la teneur totale en métal de transition est comprise entre 0,5 à 6% massique, de préférence entre 0,5 et 5% massique, et de façon encore plus préférée entre 1 et 4% massique, par rapport à la masse totale du catalyseur final, sous sa forme anhydre.

**14.** Catalyseur selon la revendication 13, ne contenant que du cuivre comme métal de transition et dans lequel la teneur massique totale en cuivre se situe entre 0,5 et 6%, de préférence 0,5 et 5%, préférablement entre 1 et 4% et de manière encore plus préférée entre 1,5 et 3,5% par rapport à la masse totale du catalyseur final sous sa forme anhydre.

**15.** Catalyseur selon la revendication 13, comprend du cuivre et un autre métal de transition, de préférence Fe, Nb, Ce, Mn, et dans lequel la teneur en cuivre se situe entre 0,05 et 2% massique, de préférence 0,5 et 2% massique, alors que la teneur de l'autre métal de transition se situe préférablement entre 1 et 4% massique, les teneurs en métaux de transition étant données en pourcentage massiques par rapport à la masse totale du catalyseur final sous sa forme anhydre.

**16.** Catalyseur selon la revendication 13, ne contenant que du fer comme métal de transition et dans lequel la teneur massique totale en fer se situe entre 0,5 et 5%, de préférence entre 1 et 4% et préférablement entre 1,5 et 3,5% par rapport à la masse totale du catalyseur final sous sa forme anhydre.

**17.** Catalyseur selon la revendication 13, comprend du fer et un autre métal de transition, de préférence Cu, Nb, Ce, Mn, et dans lequel la teneur en fer se situe entre 0,05 et 2% massique, de préférence 0,5 et 2% massique, alors que la teneur de l'autre métal de transition se situe préférablement entre 1 et 4% massique, les teneurs en métaux de transition étant données en pourcentage massiques par rapport à la masse totale du catalyseur final sous sa forme anhydre.

**18.** Utilisation du catalyseur selon l'une des revendications 13 à 17 ou préparé par le procédé selon l'une quelconques des revendications 1 à 12, pour la réduction sélective de $NO_x$ par un réducteur tel que $NH_3$ ou $H_2$.

**19.** Utilisation selon la revendication précédente, pour laquelle le catalyseur est mis en forme par dépôt sous forme de revêtement sur un monolithe, de préférence une structure nid d'abeilles ou une structure à plaques.

**20.** Utilisation selon la revendication 19, pour laquelle la structure nid d'abeilles est formée de canaux parallèles ouverts aux deux extrémités ou comporte des parois poreuses filtrantes pour lesquelles les canaux parallèles adjacents sont alternativement bouchés de part et d'autre des canaux.

**21.** Utilisation selon la revendication 20, pour laquelle la quantité de catalyseur déposé sur ladite structure est comprise entre 50 à 180 g/L pour les structures filtrantes et entre 80 et 200 g/L pour les structures avec canaux ouverts.

**22.** Utilisation selon l'une des revendications 18 à 21, pour laquelle ledit catalyseur est associé à un liant tel que la cérine, l'oxyde de zirconium, l'alumine, la silice-alumine non zéolithique, l'oxyde de titane, un oxyde mixte de type cerine-zircone, un oxyde de tungstène et/ou une spinelle, pour être mis en forme par dépôt sous forme de revêtement.

**23.** Utilisation selon l'une des revendications 18 à 22, pour laquelle ledit revêtement est associé à un autre revêtement présentant des capacités d'adsorption de NOx, de réduction des NOx ou favorisant l'oxydation de l'ammoniac.

**24.** Utilisation selon la revendication 18, pour laquelle ledit catalyseur est sous forme d'extrudé, contenant jusqu'à 100% dudit catalyseur.

**25.** Utilisation selon l'une des revendications 18 à 24, pour laquelle la structure revêtue par ledit catalyseur ou obtenue par extrusion dudit catalyseur est intégrée dans une ligne d'échappement d'un moteur à combustion interne.

**Patentansprüche**

**1.** Verfahren zur Herstellung eines Katalysators, umfassend eine Mischung von Zeolithen vom AFX-Strukturtyp und vom BEA-Strukturtyp und mindestens ein Übergangsmetall, das mindestens die folgenden Schritte umfasst:

i) Mischen eines FAU-Zeoliths mit einem $SiO_2/Al_2O_3$-Molverhältnis zwischen 30 und 100 mit mindestens einem Zeolith vom FAU-Strukturtyp mit einem $SiO_2/Al_2O_3$-Molverhältnis zwischen 2 und 30 (Obergrenze ausgeschlossen), wobei der mathematische Parameter $P_{ze}$, der dem Massenprozentanteil des FAU-Zeoliths mit einem $SiO_2/Al_2O_3$-Molverhältnis zwischen 30 und 100 in seiner wasserfreien Form (ausgedrückt in %) in der Mischung von FAU-Zeolithen multipliziert mit dem $SiO_2/Al_2O_3$-Molverhältnis des FAU-Zeoliths mit einem $SiO_2/Al_2O_3$-Molverhältnis zwischen 30 und 100 derart beschaffen ist, dass: $3250 < P_{ze} < 7200$, mindestens einer organischen Stickstoffverbindung R, ausgewählt aus 1,5-Bis-(methylpiperidinium)pentandihydroxid, 1,6-Bis-(methylpiperidinium)hexandihydroxid, 1,7-Bis-(methylpiperidinium)heptandihydroxid und Mischungen davon, und mindestens einer Quelle von mindestens einem Alkalimetall und/oder Erdalkalimetall M mit der Wertigkeit n, wobei n für eine ganze Zahl größer oder gleich 1 steht, in wässrigem Medium, wobei die Reaktionsmischung die folgende molare Zusammensetzung aufweist:

$(SiO_{2(FAU)}) / (Al_2O_{3(FAU)})$  zwischen 30 und 80,
$H_2O / (SiO_{2(FAU)})$  zwischen 1 und 100,
$R / (SiO_{2(FAU)})$  zwischen 0,01 und 0,6,

$$M_{2/n}O / (SiO_{2(FAU)}) \qquad \text{zwischen 0,005 und 0,45,}$$

wobei $SiO_{2(FAU)}$ die von allen in die Mischung eingetragenen Zeolithen vom FAU-Strukturtyp bereitgestellte molare Menge von $SiO_2$ ist, $Al_2O_{3\ (FAU)}$ die von allen in die Mischung eingetragenen Zeolithen vom FAU-Strukturtyp bereitgestellte molare Menge von $Al_2O_3$ ist, $H_2O$ die in der Reaktionsmischung vorliegende molare Wassermenge ist, R die molare Menge der organischen Stickstoffverbindung ist, $M_{2/n}O$ die von allen FAU-Zeolithen und von der Alkalimetall- und/oder Erdalkalimetallquelle bereitgestellte molare Menge von $M_{2/n}O$ ist, bis zum Erhalt eines Vorläufergels;

ii) hydrothermale Behandlung des am Ende von Schritt i) erhaltenen Vorläufergels bei einer Temperatur zwischen 120°C und 220°C über einen Zeitraum zwischen 12 Stunden und 15 Tagen zum Erhalt einer als "Feststoff" bezeichneten kristallisierten festen Phase;

iii) mindestens einen Ionenaustausch, der das Inkontaktbringen des im vorhergehenden Schritt erhaltenen Feststoffs mit mindestens einer Lösung, die mindestens eine Spezies, die mindestens ein Übergangsmetall in Lösung in reaktiver Form freisetzen kann, umfasst, unter Rühren bei Umgebungstemperatur über einen Zeitraum zwischen 1 Stunde und 2 Tagen umfasst, wobei das in der Lösung von Schritt iii) freigesetzte Übergangsmetall aus der Gruppe bestehend aus den folgenden Elementen ausgewählt ist: Ti, V, Mn, Mo, Fe, Co, Cu, Cr, Zn, Nb, Ce, Zr, Rh, Pd, Pt, Au, W, Ag, und der Gehalt an Übergangsmetall zwischen 0,5 und 6 Massen-%, bezogen auf die Gesamtmasse des fertigen Katalysators in seiner wasserfreien Form, liegt;

iv) Wärmebehandlung durch Trocknung des am Ende des vorhergehenden Schritts erhaltenen Feststoffs bei einer Temperatur zwischen 20 und 150°C über einen Zeitraum zwischen 2 und 24 Stunden und anschließende Calcinierung unter einem Luftstrom bei einer Temperatur zwischen 450 und 700 °C über einen Zeitraum zwischen 2 und 20 Stunden.

2. Verfahren nach Anspruch 1, wobei die Schritte iii) und iv) vertauscht und gegebenenfalls wiederholt werden.

3. Verfahren nach Anspruch 1 oder 2, wobei der Schritt iii) durch Inkontaktbringen des Feststoffs mit einer Lösung, die mindestens eine Spezies, vorzugsweise eine einzige Spezies, die ein Übergangsmetall freisetzen kann, umfasst, durchgeführt wird oder durch aufeinanderfolgendes Inkontaktbringen des Feststoffs mit verschiedenen Lösungen, die jeweils mindestens eine Spezies, vorzugsweise eine einzige Spezies, die ein Übergangsmetall freisetzen kann, umfassen, durchgeführt wird, wobei die verschiedenen Lösungen vorteilhafterweise unterschiedliche Spezies, die ein Übergangsmetall freisetzen können, umfassen.

4. Verfahren nach einem der Ansprüche 1 bis 3, wobei es sich bei dem in der Lösung in Schritt iii) freigesetzten Übergangsmetall um Fe, Cu, Nb, Ce oder Mn, vorzugsweise Fe oder Cu und noch weiter bevorzugt Cu handelt.

5. Verfahren nach einem der Ansprüche 1 bis 4, wobei der Gehalt an Übergangsmetall zwischen 0,5 und 5 Massen-% und noch weiter bevorzugt zwischen 1 und 4 Massen-%, bezogen auf die Gesamtmasse des fertigen Katalysators in seiner wasserfreien Form, liegt, wobei die Menge an Kupfer des Verbundwerkstoffs des Katalysators berücksichtigt wird.

6. Verfahren nach einem der Ansprüche 1 bis 5, wobei der Katalysator nur Kupfer als Übergangsmetall in einem Massengehalt zwischen 0,5 und 6 %, bevorzugt zwischen 0,5 und 5 %, vorzugsweise zwischen 1 und 4 % und noch weiter bevorzugt zwischen 1,5 und 3,5 %, bezogen auf die Gesamtmasse des fertigen Katalysators in seiner wasserfreien Form, enthält.

7. Verfahren nach einem der Ansprüche 1 bis 5, wobei der Katalysator Kupfer und ein anderes Übergangsmetall, vorzugsweise Fe, Nb, Ce, Mn, umfasst, der Kupfergehalt des erhaltenen Katalysators zwischen 0,05 und 2 Massen-%, vorzugsweise 0,5 und 2 Massen-%, liegt, während der Gehalt des anderen Übergangsmetalls vorzugsweise zwischen 1 und 4 Massen-% liegt, wobei die Übergangsmetallgehalte als Massenprozentanteile, bezogen auf die Gesamtmasse des fertigen Katalysators in seiner wasserfreien Form, angegeben sind.

8. Verfahren nach einem der Ansprüche 1 bis 5, wobei der Katalysator nur Eisen als Übergangsmetall in einem Massengehalt zwischen 0,5 und 5 %, vorzugsweise zwischen 1 und 4 % und noch weiter bevorzugt zwischen 1,5 und 3,5 %, bezogen auf die Gesamtmasse des fertigen Katalysators in seiner wasserfreien Form, enthält.

9. Verfahren nach einem der Ansprüche 1 bis 5, wobei der Katalysator Eisen und ein anderes Übergangsmetall, vorzugsweise Cu, Nb, Ce, Mn, umfasst, der Eisengehalt des erhaltenen Katalysators zwischen 0,05 und 2 Massen-%, vorzugsweise 0,5 und 2 Massen-%, liegt, während der Gehalt des anderen Übergangsmetalls vorzugsweise zwischen 1 und 4 Massen-% liegt, wobei die Übergangsmetallgehalte als Massenprozentanteile, bezogen auf die Gesamtmasse des fertigen Katalysators in seiner wasserfreien Form, angegeben sind.

10. Verfahren nach einem der vorhergehenden Ansprüche, wobei es sich bei der organischen Stickstoffverbindung R um 1,6-Bis(methylpiperidinium)hexandihydroxid handelt.

11. Verfahren nach einem der vorhergehenden Ansprüche, wobei die hydrothermale Behandlung von Schritt ii) bei einer Temperatur zwischen 120 und 220°C, bevorzugt zwischen 150 °C und 195°C, über einen Zeitraum zwischen 12 Stunden und 15 Tagen, bevorzugt zwischen 12 Stunden und 12 Tagen und noch weiter bevorzugt zwischen 12 Stunden und 8 Tagen und vorzugsweise unter autogenem Druck durchgeführt wird.

12. Verfahren nach einem der vorhergehenden Ansprüche, für den der Wärmebehandlungsschritt iv) eine Trocknung des Feststoffs bei einer Temperatur zwischen 20 und 150 °C, bevorzugt zwischen 60 und 100 °C, über einen Zeitraum zwischen 2 und 24 Stunden und eine anschließende Calcinierung, die einer Behandlung durch Verbrennung an gegebenenfalls trockener Luft entspricht, bei einer Temperatur zwischen 450 und 700 °C, vorzugsweise zwischen 500 und 600 °C, über einen Zeitraum zwischen 5 und 10 Stunden, vorzugsweise zwischen 6 und 9 Stunden, wobei die Strömungsrate von gegebenenfalls trockener Luft vorzugsweise zwischen 0,5 und 1,5 l/h/g und vorzugsweise zwischen 0,7 und 1,2 l/h/g von zu behandelndem Feststoff liegt.

13. Katalysator, umfassend einen Verbundwerkstoff, der eine innige Mischung eines Zeoliths vom AFX-Typ und eines Zeoliths vom BEA-Typ und mindestens ein Übergangsmetall enthält, mit einer Zeolithstruktur, umfassend:

- zwischen 30 und 90 Massen-%, bevorzugt zwischen 40 und 90 Massen-%, Zeolith vom AFX-Strukturtyp, bezogen auf die Gesamtmasse des Katalysators in seiner wasserfreien Form;
- zwischen 10 und 70 Massen-% Zeolith vom BEA-Strukturtyp, bevorzugt zwischen 10 und 60 Massen-%, Zeolith vom BEA-Strukturtyp, bezogen auf die Gesamtmasse des Katalysators in seiner wasserfreien Form; wobei der Katalysator ein $SiO_2/Al_2O_3$-Molverhältnis zwischen 2 und 100, bevorzugt zwischen 4 und 90 und weiter bevorzugt zwischen 6 und 80 aufweist, wobei das Übergangsmetall aus der Gruppe bestehend aus den folgenden Elementen ausgewählt ist: Ti, V, Mn, Mo, Fe, Co, Cu, Cr, Zn, Nb, Ce, Zr, Rh, Pd, Pt, Au, W, Ag, vorzugsweise Cu, Fe, Nb, Ce, Mn, sehr bevorzugt Eisen, Kupfer oder einer Mischung davon, wobei der Gesamtgehalt an Übergangsmetall zwischen 0,5 und 6 Massen-%, vorzugsweise zwischen 0,5 und 5 Massen-% noch weiter bevorzugt zwischen 1 und 4 Massen-%, bezogen auf die Gesamtmasse des fertigen Katalysators in seiner wasserfreien Form, liegt.

14. Katalysator nach Anspruch 13, der nur Kupfer als Übergangsmetall in einem Massengehalt zwischen 0,5 und 6 %, bevorzugt zwischen 0,5 und 5 %, vorzugsweise zwischen 1 und 4 % und noch weiter bevorzugt zwischen 1,5 und 3,5 %, bezogen auf die Gesamtmasse des fertigen Katalysators in seiner wasserfreien Form, enthält.

15. Katalysator nach Anspruch 13, der Kupfer und ein anderes Übergangsmetall, vorzugsweise Fe, Nb, Ce, Mn, umfasst, und bei dem der Kupfergehalt zwischen 0,05 und 2 Massen-%, vorzugsweise 0,5 und 2 Massen-%, liegt, während der Gehalt des anderen Übergangsmetalls vorzugsweise zwischen 1 und 4 Massen-% liegt, wobei die Übergangsmetallgehalte als Massenprozentanteile, bezogen auf die Gesamtmasse des fertigen Katalysators in seiner wasserfreien Form, angegeben sind.

16. Katalysator nach Anspruch 13, der nur Eisen als Übergangsmetall in einem Massengehalt zwischen 0,5 und 5 %, vorzugsweise zwischen 1 und 4 % und noch weiter bevorzugt zwischen 1,5 und 3,5 %, bezogen auf die Gesamtmasse des fertigen Katalysators in seiner wasserfreien Form, enthält.

17. Katalysator nach Anspruch 13, der Eisen und ein anderes Übergangsmetall, vorzugsweise Cu, Nb, Ce, Mn, umfasst, und bei dem der Eisengehalt zwischen 0,05 und 2 Massen-%, vorzugsweise 0,5 und 2 Massen-%, liegt, während der Gehalt des anderen Übergangsmetalls vorzugsweise zwischen 1 und 4 Massen-% liegt, wobei die Übergangsmetallgehalte als Massenprozentanteile, bezogen auf die Gesamtmasse des fertigen Katalysators in seiner wasserfreien Form, angegeben sind.

18. Verwendung des Katalysators nach einem der Ansprüche 13 bis 17 oder des durch das Verfahren nach einem der Ansprüche 1 bis 12 hergestellten Katalysators zur selektiven Reduktion von $NO_x$ durch ein Reduktionsmittel wie

NH$_3$ oder H$_2$.

**19.** Verwendung nach dem vorhergehenden Anspruch, für den der Katalysator durch Abscheidung in Form einer Beschichtung auf einem Monolith, vorzugsweise einer Wabenstruktur oder einer Plattenstruktur, gebildet ist.

**20.** Verwendung nach Anspruch 19, für die die Wabenstruktur aus an beiden Enden offenen parallelen Kanälen gebildet ist oder poröse filtrierende Wände umfasst, für die die benachbarten parallelen Kanäle auf beiden Seiten der Kanäle abwechselnd verstopft sind.

**21.** Verwendung nach Anspruch 20, für die die auf der Struktur abgeschiedene Katalysatormenge zwischen 50 und 180 g/l für die filtrierenden Strukturen und zwischen 80 und 200 g/l für die Strukturen mit offenen Kanälen liegt.

**22.** Verwendung nach einem der Ansprüche 18 bis 21, für die der Katalysator zum Formen durch Abscheidung in Form einer Beschichtung mit einem Bindemittel wie Ceroxid, Zirconiumdioxid, Aluminiumoxid, nichtzeolithischem Siliciumdioxid-Aluminiumoxid, Titandioxid, einem Ceroxid-Zirconiumdioxid-Mischoxid, einem Wolframoxid und/oder einem Spinell kombiniert ist.

**23.** Verwendung nach einem der Ansprüche 18 bis 22, für die die Beschichtung mit einer weiteren Beschichtung, die NOx-Adsorptionsvermögen oder NOx-Reduktionsvermögen aufweist oder die Oxidation von Ammoniak fördert, kombiniert ist.

**24.** Verbindung nach Anspruch 18, für die der Katalysator in Form eines Extrudats, das bis zu 100 % des Katalysators enthält, vorliegt.

**25.** Verwendung nach einem der Ansprüche 18 bis 24, für die die mit dem Katalysator beschichtete Struktur oder die durch Extrusion des Katalysators erhaltene Struktur in eine Abgasleitung eines Verbrennungsmotors integriert ist.

**Claims**

**1.** Process for preparing a catalyst comprising a mixture of AFX-structure and BEA-structure zeolites, and at least one transition metal, comprising at least the following steps:

i) mixing, in aqueous medium, of an FAU zeolite having an SiO$_2$/Al$_2$O$_3$ molar ratio of between 30 and 100 with at least one FAU-structure zeolite having an SiO$_2$/Al$_2$O$_3$ molar ratio of between 2 and 30 (upper limit excluded), and in which the mathematical parameter, P$_{ze}$, corresponding to the mass percentage of the FAU zeolite with an SiO$_2$/Al$_2$O$_3$ molar ratio of between 30 and 100, in its anhydrous form (expressed in %) in the mixture of FAU zeolites, multiplied by the SiO$_2$/Al$_2$O$_3$ molar ratio of said same FAU zeolite with an SiO$_2$/Al$_2$O$_3$ molar ratio of between 30 and 100, is such that: $3250 < P_{ze} < 7200$, of at least one nitrogenous organic compound R, chosen from 1,5-bis(methylpiperidinium)pentane dihydroxide, 1,6-bis(methylpiperidinium)hexane dihydroxide, 1,7-bis(methylpiperidinium)heptane dihydroxide, and mixtures thereof, and of at least one source of at least one alkali metal and/or alkaline-earth metal M of valence n, n being an integer greater than or equal to 1, the reaction mixture having the following molar composition:

| | |
|---|---|
| $(\text{SiO}_{2\,(FAU)})/(\text{Al}_2\text{O}_{3\,(FAU)})$ | of between 30 and 80, |
| $\text{H}_2\text{O}/(\text{SiO}_{2\,(FAU)})$ | of between 1 and 100, |
| $\text{R}/(\text{SiO}_{2\,(FAU)})$ | of between 0.01 and 0.6, |
| $\text{M}_{2/n}\text{O}/(\text{SiO}_{2\,(FAU)})$ | of between 0.005 and 0.45, |

in which SiO$_{2\,(FAU)}$ is the molar amount of SiO$_2$ provided by all the FAU-structure zeolites introduced into the mixture, Al$_2$O$_{3(FAU)}$ is the molar amount of Al$_2$O$_3$ introduced by all the FAU-structure zeolites introduced into the mixture, H$_2$O the molar amount of water present in the reaction mixture, R the molar amount of said nitrogenous organic compound, M$_{2/n}$O being the molar amount of M$_{2/n}$O provided by all the FAU zeolites and by the source of alkali metal and/or alkaline-earth metal,
until a precursor gel is obtained;
ii) hydrothermal treatment of said precursor gel obtained at the end of step i) at a temperature of between 120°C and 220°C, for a period of between 12 hours and 15 days, to obtain a solid crystalline phase, termed "solid";

iii) at least one ion exchange comprising bringing the solid obtained at the end of the previous step into contact with at least one solution comprising at least one species that is capable of releasing a transition metal, in solution in reactive form, with stirring at ambient temperature for a period of between 1 hour and 2 days, the transition metal released in the solution of step iii) being selected from the group made up of the following elements: Ti, V, Mn, Mo, Fe, Co, Cu, Cr, Zn, Nb, Ce, Zr, Rh, Pd, Pt, Au, W, Ag, and the transition metal content being between 0.5 and 6% by mass, relative to the total mass of the final catalyst, in its anhydrous form;

iv) heat treatment by drying the solid obtained at the end of the previous step at a temperature of between 20 and 150°C for a period of between 2 and 24 hours, followed by calcination under a stream of air at a temperature of between 450 and 700°C for a period of between 2 and 20 hours.

2. Process according to Claim 1, in which steps iii) and iv) are reversed, and optionally repeated.

3. Process according to Claim 1 or 2, in which step iii) is carried out by bringing the solid into contact with a solution comprising at least one species, preferably a single species, capable of releasing a transition metal or by successively bringing the solid into contact with different solutions each comprising at least one, preferably a single, species capable of releasing a transition metal, the different solutions advantageously comprising different species capable of releasing a transition metal.

4. Process according to one of Claims 1 to 3, in which the transition metal released in the solution of step iii) is Fe, Cu, Nb, Ce or Mn, preferentially Fe or Cu and even more preferably Cu.

5. Process according to one of Claims 1 to 4, in which the transition metal content is between 0.5 and 5% by mass, and even more preferably between 1 and 4% by mass, relative to the total mass of the final catalyst, in its anhydrous form, the amount of copper in the composite material of said catalyst being taken into account.

6. Process according to one of Claims 1 to 5, in which the catalyst contains only copper as transition metal, in a mass content of between 0.5 and 6%, preferably between 0.5 and 5%, preferably between 1 and 4% and even more preferably between 1.5 and 3.5% relative to the total mass of the final catalyst in its anhydrous form.

7. Process according to one of Claims 1 to 5, in which the catalyst comprises copper and another transition metal, preferably Fe, Nb, Ce, Mn, the copper content of the catalyst obtained is between 0.05 and 2% by mass, preferably between 0.5 and 2% by mass, while that of the other transition metal is preferably between 1 and 4% by mass, the transition metal contents being given as percentages by mass relative to the total mass of the final catalyst in its anhydrous form.

8. Process according to one of Claims 1 to 5, in which the catalyst contains only iron as transition metal, in a mass content of between 0.5 and 5%, preferably between 1 and 4% and even more preferably between 1.5 and 3.5% relative to the total mass of the final catalyst in its anhydrous form.

9. Process according to one of Claims 1 to 5, in which the catalyst comprises iron and another transition metal, preferably Cu, Nb, Ce, Mn, the iron content of the catalyst obtained is between 0.05 and 2% by mass, preferably between 0.5 and 2% by mass, while that of the other transition metal is preferably between 1 and 4% by mass, the transition metal contents being given as percentages by mass relative to the total mass of the final catalyst in its anhydrous form.

10. Process according to one of the preceding claims, for which the nitrogenous organic compound R is 1,6-bis(methylpiperidinium)hexane dihydroxide.

11. Process according to one of the preceding claims, in which the hydrothermal treatment of step ii) is carried out at a temperature of between 120°C and 220°C, preferably between 150°C and 195°C, for a period of between 12 hours and 15 days, preferably between 12 hours and 12 days, and more preferably between 12 hours and 8 days, and preferably under autogenous pressure.

12. Process according to one of the preceding claims, for which the heat treatment step iv) comprises drying the solid at a temperature of between 20 and 150°C, preferably between 60 and 100°C, for a period of between 2 and 24 hours, followed by calcining, corresponding to a treatment by combustion in air, which is optionally dry, at a temperature of between 450 and 700°C, preferentially between 500 and 600°C for a period of between 5 and 10 hours, preferentially between 6 and 9 hours, the flow rate of optionally dry air being preferably between 0.5 and 1.5 l/h/g, preferentially between 0.7 and 1.2 l/h/g of solid to be treated.

13. Catalyst comprising a composite material containing an intimate mixture of a zeolite of AFX type and a zeolite of BEA type, and at least one transition metal, having a zeolite structure comprising:

  - between 30 and 90% by mass, preferably between 40 and 90% by mass of AFX-structure zeolite, relative to the total mass of said catalyst in its anhydrous form;
  - between 10 and 70% by mass, preferably between 10 and 60% by mass of BEA-structure zeolite, relative to the total mass of said catalyst in its anhydrous form;
  the catalyst having an $SiO_2/Al_2O_3$ molar ratio between 2 and 100, preferably between 4 and 90 and more preferentially between 6 and 80,
  in which said transition metal is selected from the group made up of the following elements: Ti, V, Mn, Mo, Fe, Co, Cu, Cr, Zn, Nb, Ce, Zr, Rh, Pd, Pt, Au, W, Ag, preferably Cu, Fe, Nb, Ce, Mn, very preferably iron, copper or a mixture thereof,
  in which the total content of the transition metals is between 0.5 and 6% by mass, preferably between 0.5 and 5% by mass, and even more preferably between 1 and 4% by mass, relative to the total mass of the final catalyst, in its anhydrous form.

14. Catalyst according to Claim 13, containing only copper as transition metal and in which the total copper content by mass is between 0.5 and 6%, preferably between 0.5 and 5%, preferably between 1 and 4% and even more preferably between 1.5 and 3.5% relative to the total mass of the final catalyst in its anhydrous form.

15. Catalyst according to Claim 13, comprising copper and another transition metal, preferably Fe, Nb, Ce, Mn, and in which the copper content is between 0.05 and 2% by mass, preferably between 0.5 and 2% by mass, while the content of the other transition metal is preferably between 1 and 4% by mass, the transition metal contents being given as percentages by mass relative to the total mass of the final catalyst in its anhydrous form.

16. Catalyst according to Claim 13, containing only iron as transition metal and in which the total iron content by mass is between 0.5 and 5%, preferably between 1 and 4% and preferably between 1.5 and 3.5% relative to the total mass of the final catalyst in its anhydrous form.

17. Catalyst according to Claim 13, comprising iron and another transition metal, preferably Cu, Nb, Ce, Mn, and in which the iron content is between 0.05 and 2% by mass, preferably between 0.5 and 2% by mass, while the content of the other transition metal is preferably between 1 and 4% by mass, the transition metal contents being given as percentages by mass relative to the total mass of the final catalyst in its anhydrous form.

18. Use of the catalyst according to one of Claims 13 to 17 or prepared by the process according to any one of Claims 1 to 12, for the selective reduction of $NO_x$ by a reducing agent such as $NH_3$ or $H_2$.

19. Use according to the preceding claim, for which the catalyst is formed by deposition in the form of a coating on a monolith, preferably a honeycomb structure or a plate structure.

20. Use according to Claim 19, for which the honeycomb structure is formed by parallel channels open at both ends or comprises porous filtering walls for which the adjacent parallel channels are alternately blocked at both ends of the channels.

21. Use according to Claim 20, for which the amount of catalyst that is deposited on said structure is between 50 and 180 g/l for the filtering structures and between 80 and 200 g/l for the structures with open channels.

22. Use according to one of Claims 18 to 21, for which said catalyst is combined with a binder such as ceria, zirconium oxide, alumina, non-zeolitic silica-alumina, titanium oxide, a ceria-zirconia mixed oxide, a tungsten oxide and/or a spinel in order to be formed by deposition in the form of a coating.

23. Use according to one of Claims 18 to 22, for which said coating is combined with another coating having capacities to adsorb NOx, to reduce NOx, or to promote the oxidation of ammonia.

24. Use according to Claim 18, for which said catalyst is in the form of an extrudate containing up to 100% of said catalyst.

25. Use according to one of Claims 18 to 24, for which the structure coated with said catalyst or obtained by extrusion of said catalyst is integrated into an exhaust line of an internal combustion engine.

**FIG. 1**

**FIG. 2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5194235 A
- US 20160137518 A
- US 20180093259 A
- US 20160096169 A1
- US 20160096169 A
- JP 2014148441 A
- WO 2017080722 A
- WO 2017202495 A1

**Littérature non-brevet citée dans la description**

- **FICKEL, D. W. ; LOBO, R. F.** *The Journal of Physical Chemistry C,* 2009, vol. 114 (3), 1633-1640
- **LOBO, R. F. ; ZONES, S. I. ; MEDRUD, R. C.** *Chemistry of materials,* 1996, vol. 8 (10), 2409-2411
- **HRABANEK, P. ; ZIKANOVA, A. ; SUPINKOVA, T. ; DRAHOKOUPIL, J. ; FILA, V. ; LHOTKA, M. ; BERNAUER, B.** *Microporous and Mesoporous Materials,* 2016, vol. 228
- **WANG, D. et al.** *CrystEngComm.,* 2016, vol. 18 (6), 1000-1008
- **OGURA et al.** *Bull. Chem. Soc. Jpn.,* 2018, vol. 91, 355-361
- **NURIA MARTIN et al.** *Applied Catalysis B: Environmental,* 2017, vol. 217, 125-136